# EUROPEAN PATENT SPECIFICATION

(11) **EP 0 547 834 B1**
(45) Date of publication and mention of the grant of the patent: **27.05.1998**
(21) Application number: 92311230.4
(22) Date of filing: 09.12.1992
(51) Int. Cl.: C08L 23/14, C08L 23/26, C08L 51/06, C08L 53/00, C08L 23/08, A61L 2/08, C08J 7/18, B29C 71/04, B29K 23/00

(54) **Polymer blends, articles and methods for preparing same**
Polymerzusammensetzungen, Gegenständen und Verfahren zu deren Herstellung
Mélanges de polymères, articles et méthodes pour leur préparation

(30) Priority: 18.12.1991 US 809959; 28.10.1992 US 968037
(43) Date of publication of application: 23.06.1993
(73) Proprietor: MINNESOTA MINING AND MANUFACTURING COMPANY, St. Paul, Minnesota 55133-3427 (US)
(72) Inventor: Wilfong, Debra L., c/o Minnesota Mining & Manuf., St. Paul, Minnesota 55133-3427 (US); Rolando, Richard J., c/o Minnesota Mining & Manuf., St. Paul, Minnesota 55133-3427 (US)
(74) Representative: Baillie, Iain Cameron

(56) References cited:
- EP-A- 0 405 793
- EP-A- 0 431 475
- US-A- 4 401 536

## Description

This invention relates to mesopolymer blends resistant to the effects of sterilizing radiation, methods for preparing and using such polymer blends, and articles, such as fibers, films, tubes, and nonwoven fabrics formed from such polymer blends.

Medical articles or packaging for medical articles often require heat sealing in the manufacturing process to assemble the components of the article, or in the packaging process to protect the active ingredient or medical article in the packaging from undesired exposure. In addition, medical articles often require additional protection beyond secure heat sealing in manufacturing or packaging process. Specifically, these materials often need to be sterilized at the time of production and thereafter maintained in a sterile condition during storage. In this regard, a preferred method of sterilization uses gamma radiation, such as radioactive cobalt, since it can be performed on packages sealed by heat or other methods insuring total and reliable sterility of the contents.

Structural components resistant to radiation are more versatile for uses in medical articles and packaging than components unable to maintain structural integrity after irradiation. Thus, the most desirable material for a medical article or packaging is one which possesses resistance to the structurally demanding forms of sterilization even if current usages of the medical articles do not require such sterilization.

Polypropylene is often a material of choice for medical articles due to its various properties such as non-toxicity, chemical resistance and inertness to drugs and liquid media used with drugs, as well as its low cost and ease of processing by means of extrusion, molding, and the like. However, a disadvantage of crystalline polypropylene is its inherent inability to be heat sealed against another material. Furthermore, gamma irradiation of crystalline polypropylene causes degradation of its structural integrity, e.g., embrittlement, discoloration, thermal sensitivity, during or subsequent to irradiation.

In an attempt to overcome such degradation problems, various stabilizers, such as antioxidants, have been added to polypropylene homopolymers in an attempt to prevent discoloration and degradation. For example, U.S. Patent No. 4,110,185 discloses radiation sterilized articles of polypropylene which have incorporated therein a mobilizer which increases the free volume of the polymer and, therefore, lowers the density of the polymer. Suitable mobilizers mentioned include hydrocarbon oils, halogenated hydrocarbon oils, phthalic ester oils, vegetable oils, silicone oils, and polymer greases.

Also, U.S. Patent No. 4,113,595 discloses irradiated crosslinked polyolefin molded products of a blend of a polyolefin, such as polypropylene, a compound having acetylenic linkage, and an aromatic hydrocarbon-substituted organic amine or an aromatic secondary amino compound. Furthermore, U.S. Patents No. 4,274,932 and No. 4,467,065 disclose polypropylene stabilized for radiation sterilization. The polypropylene has a narrow molecular weight distribution, and has incorporated therein a mobilizer, as used in U.S. Patent No. 4,110,185, described above.

Although the addition of the various stabilizers to polypropylene homopolymers and propylene copolymers serves to diminish degradation by radiation, the use of additives increases costs. In addition, some additives may pose toxicological problems when contacted with pharmaceuticals, while other additives may adversely affect the physical properties of the polypropylene homopolymers and propylene copolymers.

In another attempt to overcome degradation problems, polyethylene has been blended with polypropylene. For example, European Patent Application No. 0068555 discloses radiation-sterilizable polypropylene-based articles, the polypropylene having one to eight weight percent of low density polyethylene added thereto. Specifically, the polyethylene is cross-linked through exposure to radiation, while the polypropylene is allowed to degrade. Thus, it is the cross-liked polyethylene that provides structural integrity for these irradiated articles.

Further attempts have been made to overcome degradation problems associated with crystalline polypropylene. For example, mesomorphous polypropylene, as described in U.S. Patent No. 4,931,230, and articles manufactured from mesomorphous polypropylene, such as described in U.S. Patent No. 4,950,549, provide resistance to sterilizing irradiation. By controlling the method of preparing mesomorphous polypropylene, such as through the quenching of such polypropylene after hot-melt extrusion, the materials or articles formed therefrom substantially maintain their structural integrity after exposure to ionizing radiation at dosages sufficient to degrade crystalline polypropylene. Furthermore, electron beam radiation can also be used to graft additional layers, such as surface adhesion promoting layers, onto films or other structures from such mesomorphous materials. See e.g., U.S. Patent No. 4,950,549.

Unfortunately, packaging films and other articles made from crystalline polypropylene, or even mesomorphous polypropylene, are also susceptible to tearing and puncturing which would disrupt the structural integrity of a manufactured component or packaging film after assembly. Thus, the usefulness of a sterilized medical article would be compromised by a puncture or tear in a polypropylene package. In addition, as noted above, crystalline polypropylene cannot be effectively heat sealed against another material. Furthermore, even though mesomorphous polypropylene provides better heat sealability than crystalline polypropylene, in certain instances it still cannot provide a sufficient heat seal to manufacture a multicomponent medical article, or to provide an effective radiation-sterilized package.

On the other hand, polybutylene, poly(1-butene) or poly(2-butene) or both, offers many advantages to the medical packaging art that polypropylene, mesomorphous or otherwise, lacks. Polybutylene has high tear strength, high impact strength, puncture resistance. Polybutylene is also often used as a film requiring heat sealability. Reference to the versatility of polybutylene may be found in Shell Technical Bulletin sc: 391-79 (1979). However, polybutylene is highly crystalline (as much as 98% crystalline). After irradiation, the melt index of polybutylene has increased, indicating chain scission. See Bradley, Journal of Industrial Irradiation Technology, (2) 93-138 (1984). Hence, polybutylene packaging usually degrades over the effective storage life often needed for a medical product. Thus, neither crystalline polypropylene nor crystalline polybutylene substantially maintain structural integrity after sterilization irradiation, while mesomorphous polypropylene lacks desirable strength packaging properties.

In an effort to overcome these deficiencies, polymer blends of mesomorphous polypropylene and a polymer compatible with such polypropylene, as described in European Patent Application No. 0 405 793 (assigned to the Assignee of the present application) have been developed. These polymer blends exhibit enhanced physical properties, such as heat sealability and tear strength, while maintaining the radiation resistance associated with mesomorphous polypropylene. However, to date, it has been assumed that propylene in its mesomorphous structure has been limited to mesomorphous polypropylene homopolymer, and a very limited number of compatible blends. In particular, it is known in the art that polypropylene and polyethylene blends are incompatible, and accordingly, the properties exhibited by such blends should follow those expected under the Rule of Mixtures.

Furthermore, it has been accepted procedure in the art to blend polypropylene copolymers with polypropylene homopolymer. The propylene in such copolymers acts as a compatibility agent with the polypropylene homopolymer. It has therefore been assumed that non-propylene containing copolymers are incompatible with polypropylene homopolymer. In addition, it has also been assumed that only the polypropylene homopolymer component of such blends is capable of assuming a mesomorphous structure.

The present invention provides mesopolymer blends, methods of forming and using such polymer blends, and articles such as films, fibers, microfibers, tubes, and pouches, that are useful for medical articles or their packaging, and that exhibit increased radiation resistance, softness, toughness, and are expected to provide good heat sealability against other materials. In addition, the surfaces of these mesopolymer blends and articles can further include additional graft layers affixed thereto by a dose of ionizing radiation.

The present invention provides a mesopolymer blend comprising an overall mesophase propylene-based material and at least one percent by weight of a blend copolymer selected from the group consisting of a mesocopolymer, an ethylene vinyl acetate copolymer, an ethylene acrylic acid copolymer, and combinations thereof, wherein said mesophase propylene-based material and said blend copolymer are different, and wherein said blend has been rapidly quenched from its melt state to provide a mesomorphic phase.

The present invention also provides a method of preparing a mesopolymer blend, comprising: (a) blending a propylene-based material and at least one percent by weight of a blend copolymer selected from the group consisting of a mesocopolymer, an ethylene vinyl acetate copolymer, an ethylene acrylic acid copolymer, and combinations thereof, and wherein said mesophase propylene-based material and said blend copolymer are different to form a polymer blend; (b) extruding the polymer blend; and (c) quenching the extruded polymer blend immediately after extrusion to provide a mesopolymer blend having an overall mesophase propylene-based material container therein, wherein the quenching occurs at a temperature of less than 38°C. In addition, this method can also comprise the further step of irradiating the mesopolymer blend with a dose of ionizing radiation capable of degrading a comparable crystalline polymer blend.

In another aspect, the present invention provides an article formed from said mesopolymer blend. Typically, such articles include films, fibers, microfibers, tubes, and pouches.

In yet another aspect, the present invention provides the use of an article formed from said mesopolymer blend comprising: (a) providing an article formed from said polymer blend; and (b) interposing the article between a protected environment and an external environment such that the protected environment remains substantially free from contamination.

For an additional appreciation of the scope of the present invention, a more detailed description of the invention follows, with reference to the drawings.

### Definitions

For the purposes of this invention the definition of "polymer" includes a homopolymer, a copolymer, or an oligomer, as well as any mixtures or blends of one or more homopolymers, and/or one or more copolymers, and/or one or more oligomers.

The term "copolymer" refers to a polymeric material produced by the polymerization of two or more dissimilar monomers, either with or without another functional group, such as maleic anhydride, grafted thereto, as well as to a homopolymer with a functional group grafted thereof. Thus, the term "copolymer" includes, without limitation, random copolymers, sequential copolymers, block copolymers, and graft copolymers.

"Propylene-based material" refers to propylene monomer, or polypropylene polymer.

"Mesophase propylene-based material", refers to a propylene-based material, in the ordered, mesophase form, which is neither amorphous, nor so ordered as to constitute isotactic I crystalline form (e.g., crystalline polypropylene) as described by G. Natta et al., "Structure and Properties of Isotactic Polypropylene", Del Nuovo Cimento, Suppl. A1, Vol. XV, Serie X, No. 1, 1960, pp. 40-51, the disclosure of which is herein incorporated by reference. A mesophase propylene-based material is formed by quenching a propylene-based material from the melt state, as defined below, and includes, without limitation, mesomorphous polypropylene and/or mesocopolymers, as those terms are defined below.

"Quenching", refers to the process of immediately and rapidly cooling a propylene-based material from the melt state such that mesophase propylene-based material is obtained.

"Mesomorphous polypropylene" (mPP) refers to polypropylene homopolymer in the mesophase form.

The term "moiety" refers to any substance which can be combined with a propylene-based material to form a copolymer, and includes, without limitation, a monomer, a polymer, or a molecule.

The term "mesocopolymer" refers to a copolymer of a propylene-based material and at least a discernable amount of at least one moiety that is quenched from the melt state to form a copolymer in the mesophase form.

A "thermoplastic copolymer" refers to a non-elastomeric, non-alicyclic, melt extrudable copolymer that is substantially free of any propylene-based material.

The term "mesopolymer blend" refers to a mixture of a mesophase propylene-based material with a discernable amount of a blend copolymer, including without limitation, mesocopolymers and/or thermoplastic copolymers.

The "Rule of Mixtures" refers to a means for determining the hypothetical values for a given physical property of a blend of two or more polymers. The hypothetical value represents the summation of the proportional contribution of the actual values of the physical property from each of the constituent polymers, based on the weight percents of the constituent polymers incorporated into the blend. Under the "Rule of Mixtures", the value for a given physical property (property "X") of a blend of two polymers (polymers A & B) can be calculated according to the following formula: Hypothetical value of property "X" for a blend of polymers A & B = (Weight percent of polymer A in the blend) x (actual value of property "X" for polymer A) + (Weight percent of polymer B in the blend B) x (actual value of property "X" for polymer B).

A "graft layer" refers to an additional layer affixed to at least a portion of a structure formed from a mesopolymer blend of the present invention by grafting a compound or compounds to the surface of the structure through the application of a dosage of ionizing radiation, preferably a dosage of electron beam radiation. For example, a graft layer of acrylic acid (AA) and/or dimethylacrylamide (DMA) can be grafted to at least a portion of the surface of the mesopolymer blend structures of the present invention through the exposure of such compound(s) and structures to a dosage of electron beam radiation between about 5 kGy (0.5 Mrad) and about 200 kGy (20 Mrad). In such an instance, the grafted acrylic acid and/or dimethylacrylamide layer would form a surface adhesion layer that promotes the adhesion of other materials to the modified surface of the structure. Such modification of the surface properties of a structure formed from a mesopolymer blend is to be contrasted with the bulk properties attributable to, and as a result of, the quenching of a copolymer from the melt state to form a mesocopolymer.

A "compatible copolymer" refers to any blend copolymer, such as a mesocopolymer and/or a thermoplastic copolymer, which when combined with mesophase propylene-based material forms a mesopolymer blend wherein at least one weight fraction of the mesopolymer blend has a more desirable physical property than would be expected under the Rule of Mixtures.

The "structural integrity" of a mesopolymer blend can be measured by the percent elongation to break of a structure (expressed as fracture strain percent), such as a film, formed from the mesopolymer blend. With respect to radiation resistance of such structures, percent elongation to break is used to measure the extent of degradation or embrittlement of these structures after exposure to radiation. A substantially constant percent elongation at break over several months after irradiation is indicative of substantial maintenance of structural integrity of a mesopolymer blend structure over that period after irradiation.

### Brief Description of the Drawings

The invention may be further illustrated by reference to the accompanying Drawings wherein:
FIG. 1 is the wide-angle x-ray diffraction pattern of mesomorphous polypropylene (Example 1);
FIG. 2 is the wide-angle x-ray diffraction pattern of crystalline polypropylene (Comparative Example 18);
FIG. 3 is the wide-angle x-ray diffraction pattern of a 50/50 mesopolymer blend by weight of mesomorphous polypropylene with ethylene vinyl acetate copolymer (Example 3);
FIG. 4 is the wide-angle x-ray diffraction pattern of a 50/50 polymer blend by weight of crystalline polypropylene with ethylene vinyl acetate copolymer (Comparative Example 20);
FIG. 5 is the wide-angle x-ray diffraction pattern of 50/50 mesopolymer blend by weight of mesomorphous polypropylene with polybutylene (Example 6);
FIG. 6 is the wide-angle x-ray diffraction pattern of a 50/50 polymer blend by weight of crystalline polypropylene with polybutylene (Comparative Example 23);
FIG. 7 is the wide-angle x-ray diffraction pattern of a 25/25/50 mesopolymer blend by weight of mesomorphous polypropylene, polybutylene, and ethylene vinyl acetate copolymer (Example 8);
FIG. 8 is the wide-angle x-ray diffraction pattern of an 25/25/50 polymer blend by weight of crystalline polypropylene, polybutylene, and ethylene vinyl acetate copolymer (Comparative Example 25);
FIG. 9 is the wide-angle x-ray diffraction pattern of a 50/50 mesopolymer blend by weight of mesomorphous polypropylene with ethylene acrylic acid copolymer (Example 11);
FIG. 10 is the wide-angle x-ray diffraction pattern of a 50/50 polymer blend by weight of crystalline polypropylene with ethylene acrylic acid copolymer (Comparative Example 28);
FIG. 11 is the wide-angle x-ray diffraction pattern of a 25/25/50 mesopolymer blend by weight of mesomorphous polypropylene, polybutylene, and ethylene acrylic acid copolymer (Example 14);
FIG. 12 is the wide-angle x-ray diffraction pattern of an 25/25/50 polymer blend by weight of crystalline polypropylene, polybutylene, and ethylene acrylic acid copolymer (Comparative Example 31);
FIG. 13 is the wide-angle x-ray diffraction pattern of a 50/50 mesopolymer blend by weight of a polypropylene/polyethylene mesocopolymer (CO1) with another polypropylene/polyethylene mesocopolymer (CO2) (Example 15);
FIG. 14 is the wide-angle x-ray diffraction pattern of a 50/50 polymer blend by weight of a polypropylene/polyethylene copolymer (CO1) with another polypropylene/polyethylene copolymer (CO2) (Comparative Example 32);
FIG. 15 is the wide-angle x-ray diffraction pattern of a 50/50 mesopolymer blend by weight of mesomorphous polypropylene with a polypropylene/polyethylene mesocopolymer (CO1) (Example 16);
FIG. 16 is the wide-angle x-ray diffraction pattern of a 50/50 polymer blend by weight of crystalline polypropylene with a polypropylene/polyethylene copolymer (CO1) (Comparative Example 33);
FIG. 17 is the wide-angle x-ray diffraction pattern of a 50/50 mesopolymer blend by weight of ethylene vinyl acetate copolymer with a polypropylene/polyethylene mesocopolymer (CO1) (Example 17);
FIG. 18 is the wide-angle x-ray diffraction pattern of a 50/50 polymer blend by weight of ethylene vinyl acetate copolymer with a polypropylene/polyethylene copolymer (CO1) (Comparative Example 34);
FIG. 19 is a graph comparing the radical decay as measured in normalized radical peak height in spins/gram as a function of elapsed time in hours for the mesopolymer blend film of Example 2 (line A) and for the crystalline polymer blend film of Comparative Example 19 (line B) after exposure to a 50 kGy dosage of electron beam radiation;
FIG. 20 is a graph comparing the radical decay as measured in normalized radical peak height in spins/gram as a function of elapsed time in hours for the mesopolymer blend film of Example 3 (line A) and for the crystalline polymer blend film of Comparative Example 20 (line B) after exposure to a 50 kGy dosage of electron beam radiation;
FIG. 21 is a graph comparing the radical decay as measured in normalized radical peak height in spins/gram as a function of elapsed time in hours for the mesopolymer blend film of Example 7 (line A) and for the crystalline polymer blend film of Comparative Example 24 (line B) after exposure to a 50 kGy dosage of electron beam radiation;
FIG. 22 is a graph comparing the radical decay as measured in normalized radical peak height in spins/gram as a function of elapsed time in hours for the mesopolymer blend film of Example 10 (line A) and for the crystalline polymer blend film of Comparative Example 27 (line B) after exposure to a 50 kGy dosage of electron beam radiation;
FIG. 23 is a graph comparing the radical decay as measured in normalized radical peak height in spins/gram as a function of elapsed time in hours for the mesopolymer blend film of Example 11 (line A) and for the crystalline polymer blend film of Comparative Example 28 (line B) after exposure to a 50 kGy dosage of electron beam radiation;
FIG. 24 is a graph comparing the radical decay as measured in normalized radical peak height in spins/gram as a function of elapsed time in hours for the mesopolymer blend film of Example 13 (line A) and for the crystalline polymer blend film of Comparative Example 30 (line B) after exposure to a 50 kGy dosage of electron beam radiation;
FIG. 25 is a graph comparing fracture strain (elongation at break) of the mesopolymer blends of Examples 1-5 (line A) with hypothetical linear values for Examples 1-5 (line B);
FIG. 26 is a graph comparing the fracture strain of the mesopolymer blends of Examples 5-9 (line A) with hypothetical linear values for Examples 5-9 (line B);
FIG. 27 is a graph comparing the fracture strain of the mesopolymer blends of Examples 1, 10-12 and 35 (line A) with hypothetical linear values for Examples 1, 10-12 and 35 (line B); and
FIG. 28 is a graph the fracture strain of the mesopolymer blends of Examples 6, 13-14 and 35-36 (line A) with hypothetical linear values for Examples 6, 13-14 and 35-36 (line B).

### Detailed Description of Embodiments of the Invention

The mesopolymer blends of the present invention are capable of substantially maintaining their structural integrity for a useful period after irradiation and provide other enhanced properties, such as lower modulus of elasticity and increased fracture strain. Furthermore, it is expected that the mesopolymer blends of the present invention will exhibit many, if not all, of the same advantages, including toughness, heat-sealability, and/or quietness, as barrier structures formed with mesomorphous polypropylene, mesocopolymers, and other structures formed therefrom. See, e.g., Applicants' copending and co-filed U.S. Patent Applications Serial Number 07/810001, and Attorney Docket Nos. 47008USA3A and 47008USA1B, Wilfong et al., and 47990USA2A and 47990USA1B, Rolando et al., all assigned to the Assignee of the present invention, and the disclosures of which are herein incorporated by reference.

The mesopolymer blends are produced from a mesophase propylene-based material (i.e. mesomorphous polypropylene (mPP) and/or a mesocopolymer) blended with a blend copolymer. Nonlimiting classes of blend copolymers include mesocopolymers, thermoplastic copolymers compatible with the mesophase propylene-based material, such as ethylene vinyl acetate copolymer (EVA), ethylene acrylic copolymer (EAA), and combinations thereof, as well as polybutylene (PB) with EVA, PB with EAA, and combinations of PB with EVA and EAA. Thus, blends of mPP with a mesocopolymer, EVA, EAA, and/or PB, as well as blends of a mesocopolymer with another mesocopolymer, EVA, EAA, and/or PB, are all considered to be within the scope of the present invention.

The particular combination of blend copolymer(s) with a selected mesophase propylene-based material will depend upon the desired properties of, and intended use for, the ultimate structures formed from the mesopolymer blends of the present invention. For example, in one embodiment, the blend copolymer comprises a mesocopolymer, such as a polypropylene/polyethylene block copolymer, that can surprisingly form a mesophase structure alone, as well as in a mesopolymer blend with a mesophase propylene-based material, such as mesomorphous polypropylene or another mesocopolymer. In another embodiment, the blend copolymer will comprise a thermoplastic copolymer, such as EVA, that is compatible with the mesomorphous propylene-based material component of the mesopolymer blend. In a preferred embodiment, both the selected mesocopolymer and/or thermoplastic copolymer will be compatible with the mesophase propylene-based material.

As described above, compatibility of a copolymer with a mesophase propylene-based material is determined by a comparison of one or more of the physical properties of a weight fraction of a blend with the hypothetical value of the same physical property(s) at that weight fraction as determined by the respective blended mesophase propylene-based material and copolymers in their proportionate weight fraction. Typically, percent elongation to break, as measured in fracture strain percent, is a good indicator of the compatibility of a blend copolymer with a mesophase propylene-based material. However, it is within the scope of the present invention to provide blend copolymers, such as mesocopolymers, that are not compatible with the mesophase propylene-based material, but provide unique mesomorphous structures that display one or more advantageous properties, such as increased softness, toughness, radiation resistance, and quietness. Thus, mesopolymer blends of a first mesocopolymer with a second mesocopolymer and/or mesomorphous polypropylene may occur that are not compatible, but are nonetheless unknown to those skilled in the art, and display one or more of the advantageous properties noted above.

The optimum weight fraction of the blend copolymer with a mesophase propylene-based material depends upon the ultimate intended use of the mesopolymer blend and the desired properties. Generally, it is desirable to add as much blend copolymer as possible to provide strength, heat sealability, and other packaging properties without compromising the radiation resistance provided by the mesophase propylene-based material.

However, it is within the scope of this invention to add a discernibly minimal amount of the blend copolymer to the mesophase propylene-based material to provide a mesopolymer blend, quenched to preserve mesomorphous structure, having excellent sterilization irradiation resistance, and better physical properties, such as elongation at break and softness, than the mesophase propylene-based material alone. Furthermore, if the blend copolymer comprises a thermoplastic copolymer, and if the resulting mesopolymer blend having a minimal weight fraction of the thermoplastic copolymer can be discerned to have a greater percent elongation at break value, or other physical property value, than the hypothetical linear value computed according to the equation described in the Definitions above, then the mesopolymer blend is within the scope of this invention.

Optionally, as little as one percent by weight of the blend copolymer to the weight of the mesopolymer blend, quenched to preserve mesomorphous structure, may form an acceptable mesopolymer blend for certain medical packaging devices to provide a radiation resistant blend having some desirable packaging properties.

It is also within the scope of this invention to add a discernibly minimal amount of propylene-based material to the blend copolymer to provide a blend, quenched to preserve mesomorphous structure, having excellent medical packaging properties and acceptable radiation resistance. Analogously, if the mesopolymer blend having a minimal weight fraction of mesophase propylene-based material in combination with a thermoplastic copolymer can be discerned to have a greater percent elongation at break value, or other physical property value, than the hypothetical linear value computed according to the equation described in the Definitions above, then the mesopolymer blend is within the scope of this invention.

Optionally, as much as ninety-nine percent (99%) by weight of the blend copolymer to the weight of the mesopolymer blend, may form an acceptable mesopolymer blend for certain medical packaging purposes.

Desirably, the weight fraction range of the blend copolymer is from about five percent (5%) to about ninety-five percent (95%) by weight, and more desirably from about ten percent (10%) to about ninety percent (90%) by weight of the mesopolymer blend.

Preferably, for balance of the best properties of the mesophase propylene-based material and the blend copolymer in the mesopolymer blend, a weight fraction of the blend copolymer ranges from about twenty percent (20%) to about eighty percent (80%) by weight, more preferably from about twenty-five (25%) to about seventy-five percent (75%) by weight, and most preferably from about forty percent (40%) to about sixty percent (60%) by weight of the mesopolymer blend.

When a mesocopolymer is employed in the mesopolymer blends of the present invention, virtually any moiety or combination of moieties can be used in conjunction with propylene-based materials to form the mesocopolymer component. Prior to melt extrusion and quenching, the propylene-based material and other moiety or moieties can be combined in any percent by weight ratio which will result upon quenching in the mesocopolymer. Preferably, the optimum weight fraction of propylene-based material with the other moiety depends upon the intended use and desired properties of the mesocopolymer component of the mesopolymer blend. Generally, it is desirable to add as much of the other moiety as possible, without compromising the overall mesophase form and its advantageous properties, such as softness and radiation resistance. For a more detailed review of preferred weight fractions of propylene-based materials and moieties in the mesocopolymers, reference should be had to Applicants' European Patent document EP-A-547824.

The mesocopolymers useable to form the mesopolymer blends of the present invention generally fall within three classes. The first class of copolymer comprises a mesocopolymer wherein the other moiety comprises a monomer, such as ethylene or butylene, that is inserted with propylene monomers in a copolymer chain. Accordingly, class one mesocopolymers of the present invention include, without limitation, random, sequential, and block copolymers. A commercially available example of such a copolymer, which when quenched forms a mesocopolymer according to the present invention, is Petrothane® resin No. PP7300-KF (Quantum Chemical, Inc.). However, it is contemplated that any copolymer that can be formed, for example by a catalyzed or photo-initiated polymerization reaction, melted, and quenched to preserve a mesophase structure, is considered to be within the scope of the present invention.

The second class of mesocopolymers useable in the mesopolymer blends of the present invention comprise quenched copolymers of the above described class one copolymers, with another moiety grafted to the copolymer chain. For example, the other moiety can comprise a functional group, such as maleic anhydride or acrylic acid, grafted to the copolymer chain, to provide enhanced melt flow rates, as well as other bulk properties. See, e.g., U.S. Patent No. 4,003,874, and British Patent No. 1,393,693, the disclosures of which are herein incorporated by reference. A commercially available example of such a copolymer is Plexar® resin No. 420 (Quantum Chemical, Inc.).

The third, and final, general class of copolymers, which when quenched can provide mesocopolymers, comprise a polypropylene homopolymer with a moiety, such as maleic anhydride or acrylic acid, grafted to the polymer chain. A commercially available example of such a copolymer is Admer® resin No. QF551A (Mitsui Plastics, Inc.).

In a preferred embodiment, the mesocopolymer component of the mesopolymer blends of the present invention comprises a class one copolymer of a propylene monomer combined with a discernable amount of at least one other monomer to form a random, sequential or block copolymer, that is melted and quenched to preserve the mesophase structure. Preferably, the propylene monomer will comprise from about 1% to about 99%, more preferably from about 50% to about 99%, and most preferably from about 90% to about 99% by weight of the mesocopolymer, with the remainder comprising the other monomer, or monomers. The monomers to be combined with propylene to form the mesocopolymers can include any monomer that would polymerize with propylene in the presence of a suitable catalyst, including ethylene, butylene, pentene, methylpentene, and the like. Preferred monomers include ethylene and butylene, with ethylene being particularly preferred.

In a particularly preferred embodiment, the mesocopolymers comprise copolymers of an ethylene monomer with a propylene monomer, quenched to provide the mesocopolymer. Preferably, the ethylene monomer comprises from about 1% to about 99%, more preferably from about 1% to about 25%, more preferably yet from about 1% to about 20%, and most preferably from about 1% to about 10% by weight of the mesocopolymer, with the remaining monomer comprising propylene.

Many commercially available propylene-based copolymers which when quenched from the melt state could serve to form mesocopolymer component of the mesopolymer blends according to the present invention. Nonlimiting examples of such commercially available propylene-based copolymers include Admer® resin No. QF551A, a polypropylene graft copolymer (Mitsui Plastics, Inc.; melt index = 5.7 g/10 min); Plexar® resin No. 420, a polypropylene/polyethylene graft copolymer (Quantum Chemical Corp.; melt index = 2.5 g/10 min.); Shell resin No. 7C05N, a 40% to 60% by weight polypropylene/polyethylene (PP/PE) copolymer combined at 14% by weight with 86% by weight of polypropylene homopolymer (Shell Chemical Corp.; melt index = 15 g/10 min.); PP/PE copolymer resin No. 6571 (Fina Oil and Chemical Co.; melt index = 8 g/10 min.; low ethylene content; melting point (MP) = 148°C); PP/PE copolymer resin No. 7371 (Fina; melt index = 3.5 g/10 min.; medium ethylene content; MP = 143°C); PP/PE copolymer resin No. 8473 (Fina; melt index = 4.6 g/10 min.; high ethylene content; MP = 134°C); and PP/PE copolymer resin No. Z9350 (Fina; melt index = 3.5 g/10 min.; random copolymer; MP = 129°C).

Totally optionally, to provide specific additional properties, the mesopolymer blends of the present invention can be extruded with other conventional additives such as antistatic materials, pigments, dyes, plasticizers, ultraviolet absorbers, nucleating agents, quenching agents such as mineral oil, and the like. The amount of these materials is typically less than ten percent (10%) by weight of the mesopolymer blend, and preferably less than two percent (2%) by weight of the blend. However, the blends do not require any stabilizers, anti-oxidants or the like to enable the mesopolymer blend to withstand the effects of ionizing radiation and substantially maintain structural integrity for a useful period after irradiation.

In addition, the outer surfaces of structures, such as films, formed from the mesopolymer blends of the present invention may also desirably have one or more graft layers affixed thereto to enhance other properties, such as surface adhesion, oxygen and/or moisture permeability, coefficient of friction, or other properties desirable to those skilled in the art. For example, not be way of limitation, surface adhesion is desirable in order to provide the application of primers and other coatings that would not otherwise adhere well to the structures of the present invention. In this regard, modification of surface properties of a structure formed from mesopolymer blends of the present invention is different and distinct from the bulk properties, such as increased softness and radiation resistance, attributable to mesocopolymers due to their melting and quenching according to the methods of the present invention. Thus, the melting and quenching of a propylene graft copolymer, such as Admer® resin No. QF551A, to provide a mesocopolymer with increased softness and radiation resistance throughout its mass, and useful as a component of the mesopolymer blends of the present invention, is to be contrasted from the modification of the surface of an article formed from a mesopolymer blend, by radiation grafting an additional compound or compounds thereon.

In a preferred embodiment, a graft layer, such as a surface adhesion layer, is grafted to the outer surfaces of structures formed from mesopolymer blends by electron-beam radiation at dosages of from about 5 kGy (0.5 Mrad) to about 200 kGy (20 Mrad), and preferably at about 50 kGy (5 Mrad), according to the procedures provided in U.S. Patent No. 4,950,549, the disclosure of which is herein incorporated by reference. Nonlimiting examples of compounds that can be grafted to these mesopolymer blend structures of the present invention to form a surface adhesion layer include acrylic acid (AA), dimethylacrylamide (DMA), N-vinyl-2-pyrrolidone (NVP), and a copolymer of NVP and trimethylolpropanetriacrylate (NVP/TMPTA). Other potential compounds that can also be used as a graft layer include glycidyl acrylate, hydroxyethyl acrylate, hydroxymethyl acrylate, 2-vinyl pyridine, sulfoethyl methacrylate, diisopropylacrylamide, or N,N-diethylamino acrylate. Particularly preferred grafting compounds used to form a surface adhesion layer on a least a portion of these mesopolymer blend structures according to the present invention include AA (Aldrich Chemical Co., Milwaukee, WI) and DMA (Chem Services, Inc., Westchester, PA).

The mesopolymer blends according to the present invention are substantially softer than comparable non-quenched crystalline polymer blends. Specifically, the mesopolymer blends exhibit a modulus of elasticity (i.e. Young's modulus measured in Mega Pascals (MPa)) from about one percent to about ninety nine percent less, preferably from about five percent to about fifty percent less than a comparable crystalline structure. In addition the mesopolymer blends of the present invention also exhibit increased radiation resistance, and increased toughness (as measured by the fracture strain) across several weight fractions, and are expected to exhibit increased quietness (as measured in Hertz (Hz) of sound emitted), when compared to crystalline propylene-based polymer blends. Specifically, it is expected that the mesopolymer blends of the present invention can withstand ionizing radiation dosages of from about 1 kGy (0.1 Mrad) to 200 kGy (20.0 Mrad), and still maintain structural integrity for an extended period of time.

Although mesomorphous polypropylene and a very limited number of compatible polymer blends are known (Natta, G., et al. Structure and Properties of Isotactic Polypropylene, Del Nuovo Cimento Supplemento Al, Volume XV, Serie X, N.1, 1960, pp. 40-51; EPO Application No. 0405793), the present invention provides mesopolymer blends, and articles manufactured therefrom, which were not previously thought to be possible to form with a mesophase structure. Specifically, the art teaches that polypropylene and polyethylene blends are incompatible, i.e. that the properties of the resulting blends follow those that would be expected under the Rule of Mixtures. However, surprisingly, the mesopolymer blends of propylene-based materials with compatible thermoplastic copolymers such as EVA and EAA (and also with PB) according to the present invention exhibit synergy in fracture strain above what would be expected of such blends under the Rule of Mixtures. Furthermore, it has also been unexpectedly discovered that mesopolymer blends, wherein the mesophase propylene-based material and/or the compatible copolymer comprises a mesocopolymer, can be formed such that the mesocopolymer component, in and of itself, displays mesophase structure. Thus, it is possible according to the present invention to form mesopolymer blends, wherein one or more components of blend comprise copolymers that exhibit mesophase structure. To date, the existence of mesocopolymers singly, let alone in a mesopolymer blend, has been unknown.

While not being held to a theory of operation, it is suspected that the radiation stability of the mesopolymer blends of the present invention, as well as the mesocopolymer and/or mPP components of such blends, is related to the control of their morphology. Mesomorphous polypropylene has been described as a non-spherulitic structure by P.H. Geil (Polymer Single Crystals, Interscience, N.Y., 1963, p. 270). Conversely, crystalline polypropylene may have "chain-folds", i.e., crystalline/amorphous folds, in the structure which provide areas for radical attack because of their higher energy. In contrast, mesophase structure, such as is present in the mesopolymer blends of the present invention, is believed to have ordering as in a Fringed Micelle model, i.e. with no chain-fold defects. It is suspected that this lack of chain fold defects minimizes the number of sites for radical attack and thereby provides the resistance to radiation degradation observed in the mesopolymer blends of the present invention.

The process of blending known mixtures of polymers and copolymers is well known to those skilled in the art. The available methods of blending are well described in the literature, such as, Mathews, Polymer Mixing Technology, Chapter 3 (Applied Science Publishers, Essex, England, 1982), the disclosure of which is incorporated herein by reference. In the case of the present invention, the method of blending involves the use of an extruder by feeding the polymers and/or copolymers (in the proper weight percentages) that have been dry-blended together prior to the melt extrusion process.

The various polymer blends can be extruded from the polymer melt in any shape which can be rapidly cooled to obtain the mesopolymer blends. The shape and/or thickness of the extruded material will be dependent on the efficiency of the quenching systems utilized. Generally, films, fibers, tubes, and blown microfiber webs are the preferred extruded materials. However, the extruded mesopolymer blends should not be subjected to temperature treatment that would change the mesophase form of the mesopolymer blends to the crystalline phase. After irradiation, the mesopolymer blends can be stretched or oriented if properties provided by such treatment are desired.

To obtain a mesopolymer blend having mesophase propylene-based materials contained therein, the extruded blend must be quenched in a manner such that primarily the mesomorphous phase of the propylene-based material is obtained. Miller, "On the Existence of Near-Range Order in Isotactic Polypropylenes," in Polymer, One 135 (1960), and U.S. Patent No. 4,931,230, the disclosures of which are both incorporated by reference herein, disclose suitable methods known to those skilled in the art for preparation of mesomorphous polypropylene. In this regard, various known methods of quenching as soon as possible, and preferably immediately after extrusion can be used to obtain a mesopolymer blend having mesophase propylene-based materials therein, including plunging the extruded material into a cold liquid, e.g., ice water bath, spraying the extruded material with a liquid such as water, and/or running the extruded material over a cooled roll or drum.

In a preferred embodiment, the mesopolymer blends of the present invention comprise a film that is preferably quenched immediately after extrusion by contact with a quench roll or by plunging the film into a quench bath. For a film thickness of from about 0.025 mm to about 0.25 mm, where a quench roll is used, roll temperature is preferably maintained at a temperature below about 38°C, more preferably at a temperature below 24°C, and the film is generally in contact with the roll until solidified. The quench roll should be positioned relatively close to the extruder die, the distance being dependent on the roll temperature, the extrusion rate, the film thickness, and the roll speed. Generally, the distance from the die to the roll is about 0.25 cm to about 5 cm. Where a quench bath is used, the bath temperature is preferably maintained at a temperature below about 4°C. The bath should be positioned relatively close to the die, generally about 0.25 cm to about 13 cm from the die to the bath.

In another preferred embodiment, the mesopolymer blends comprise melt blown microfibers that are produced by extruding molten polymer through a die into a high velocity hot air stream to produce fibers having an average fiber diameter of less than about 10 microns. The fibers are collected on a drum in the form of a web. The preparation of microfiber webs is described in Report No. 4364 of the Naval Research Laboratories, published May 25, 1954, entitled "Manufacture of Superfine Organic Fibers," by Wente, Van A. et al. and in Wente, Van A., "Superfine Thermoplastic Fibers" in Industrial Engineering Chemistry, Vol. 48, No. 8, August, 1956, pp. 1342-1346, the disclosures of which are both herein incorporated by reference.

To achieve webs of the mesopolymer blends of the invention, the blown microfiber web is preferably quenched by spraying with a liquid such as water or by cooling the collector drum onto which the microfiber web is collected. Optimum quenching can be achieved by spraying the fiber web near the die, then collecting the web on a cooled drum. The water spray is preferably at a temperature of less than about 10°C and less than about 2.5 cm from the die and the collector drum is preferably about 5 cm to about 10 cm from the die, but can be as much as about 20 cm to about 25 cm depending on extrusion rates.

In addition, other useful articles such as tapes, tubings, containers, transdermal drug-delivery patches and various packaging materials can also be formed from the mesopolymer blends according to the present invention. Thus, articles useful to form or cover a protective environment from an external environment, such that the protected environment remains substantially free of contamination when protecting a degradable product contained therein, or a surface covered thereby. For example, such articles can be used to contain a food product or a pharmaceutical product in a protected environment. Similarly, these articles can comprise a transdermal drug-delivery patch, medical tape, medical tubing, or an ostomy pouch, and can protect the body of a mammal, or contain the bodily fluids or waste products of the mammal from degradation due to exposure to the external environment.

The following non-limiting examples are provided to further illustrate the invention.

### EXAMPLES 1-17, 35-36, AND COMPARISON EXAMPLES 18-34

Thirty-six, single-layer extruded films were made using a flat film process. The films of Examples 1-14, 35-36, and Comparative Examples 18-31 were comprised of mixtures by weight of polypropylene homopolymer (PP1) (resin No. PP3576; Fina Oil and Chemical Co.; melt index = 9 g/10 min.) with ethylene vinyl acetate copolymer (EVA) (resin No. UE656; Quantum Chemical Corporation; 12% vinyl acetate comonomer, melt index = 5.4 g/10 min.), or with ethylene acrylic acid copolymer (EAA) (PRIMACOR® resin No. 3340; Dow Chemical Company; 6.5% acrylic acid comonomer; melt index = 9 g/10 min.), or with both EVA and polybutylene homopolymer (PB) (resin No. PB400; Shell Chemical Co.; melt index = 20 g/10 min.), or with both EAA and PB. The films of Examples 15-17 and Comparative Examples 32-34 were comprised of mixtures by weight of another polypropylene homopolymer (PP2) (resin No. 5A95; Shell Chemical Co.; melt index = 9 g/10 min.) or ethylene vinyl acetate copolymer (EVA) (resin No. UE656; Quantum Chemical Corp.) with a polypropylene/polyethylene copolymer (CO1) (resin No. 7371; Fina Oil and Chemical Co.; medium ethylene content; melt index = 3.5 g/10 min.), or a mixture of CO1 with a second polypropylene/polyethylene copolymer (CO2) (resin No. 6571; Fina Oil and Chemical Co.; low ethylene content; melt index = 8 g/10 min.). Blend mixtures were dry blended before being melted and extruded.

The films of Examples 1-14, 35-36, and Comparative Examples 18-31 were extruded at a melt temperature of 220°C, while the films of Examples 15-17 and Comparative Examples 32-34 were extruded at a melt temperature of 260°C. The temperature for the quench roll for Examples 1-17 and 35-36 was maintained at 10°C or 18°C in order to control the mesomorphous structure (meso) of the polypropylene in the mesopolymer blend films, and at 66°C for Comparison Examples 18-34 to produce crystalline structured (crys) polypropylene in the blend films. Films were generally extruded at a film thickness of about 0.05 mm to about 0.075 mm.

Polymer blend composition, blend ratio (Weight percent), Wide Angle X-Ray Diffraction (WAXD) structure (i.e. mesomorphous = meso; crystalline = crys), and casting roll temperature for Example films 1-17, and Comparative Example films 18-34 are shown in Table 1. In addition to the films shown in Table 1, two additional Examples films were formed. Example film 35 comprised a one hundred percent (100%) EAA copolymer film, and Example 36 comprised a PP:PB:EAA film at a 1:1:6 weight ratio.

**Table 1**

| Polymer blend composition, blend ratio (Weight percent), WAXD structure, and casting roll temperature for Example films 1-17, and Comparative Example films 18-34. | | | | |
|---|---|---|---|---|
| Example No./ Comparative Example No. | Polymer Blend Composition | Blend Ratio (Wt%) | WAXD Structure | Casting Roll Temp. (°C) |
| 1/18 | PP1:EVA | 1:0 | meso/crys | 10/66 |
| 2/19 | PP1:EVA | 3:1 | meso/crys | 10/66 |
| 3/20 | PP1:EVA | 1:1 | meso/crys | 10/66 |
| 4/21 | PP1:EVA | 1:3 | meso/crys | 10/66 |
| 5/22 | PP1:EVA | 0:1 | na | 10/66 |
| 6/23 | PP1:PB:EVA | 1:1:0 | meso/crys | 10/66 |
| 7/24 | PP1:PB:EVA | 3:3:2 | meso/crys | 10/66 |
| 8/25 | PP1:PB:EVA | 1:1:2 | meso/crys | 10/66 |
| 9/26 | PP1:PB:EVA | 1:1:6 | meso/crys | 10/66 |
| 10/27 | PP1:EAA | 3:1 | meso/crys | 10/66 |
| 11/28 | PP1:EAA | 1:1 | meso/crys | 10/66 |
| 12/29 | PP1:EAA | 1:3 | meso/crys | 10/66 |
| 13/30 | PP1:PB:EAA | 3:3:2 | meso/crys | 10/66 |
| 14/31 | PP1:PB:EAA | 1:1:2 | meso/crys | 10/66 |
| 15/32 | CO1:CO2 | 1:1 | meso/crys | 18/66 |
| 16/33 | PP2:CO1 | 1:1 | meso/crys | 18/66 |
| 17/34 | EVA:CO1 | 1:1 | meso/crys | 18/66 |

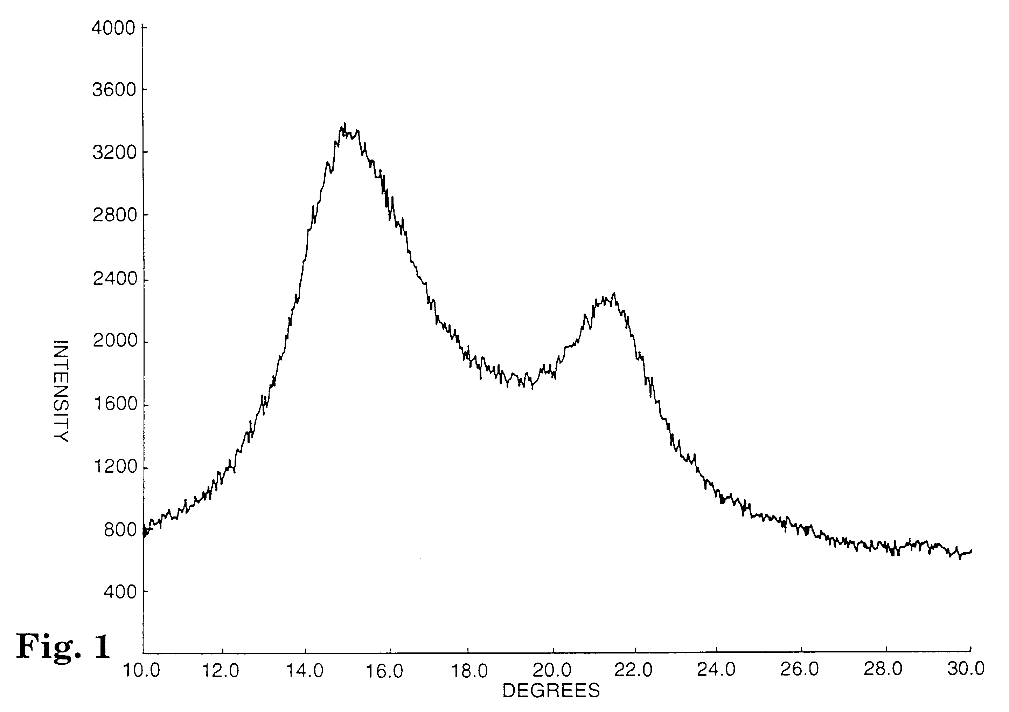

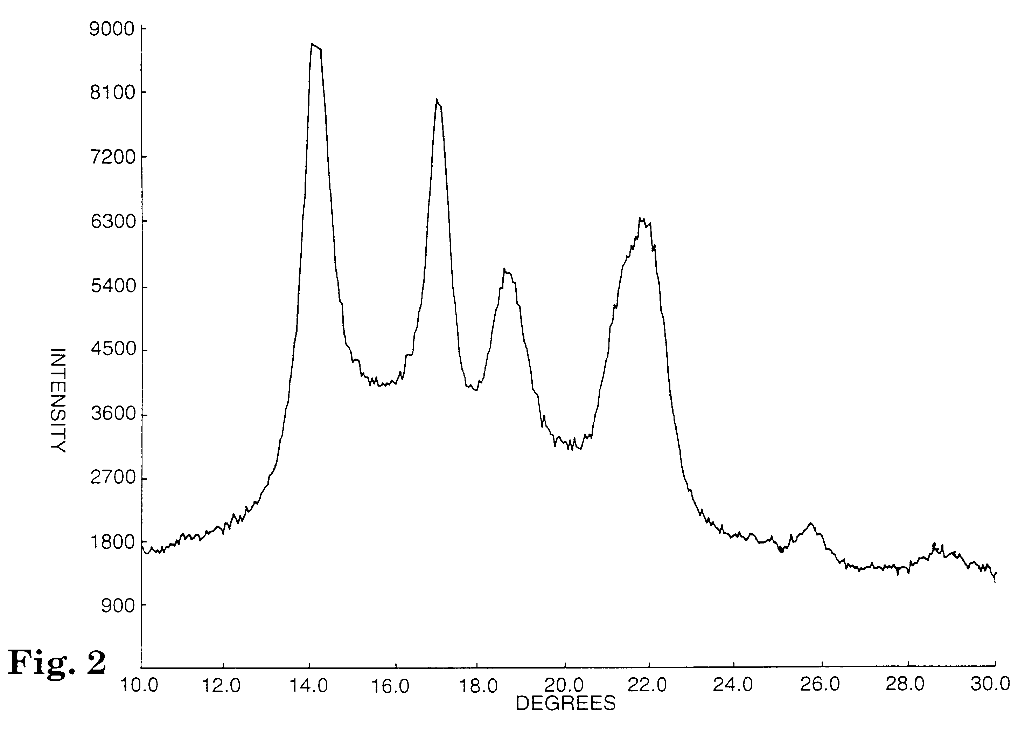

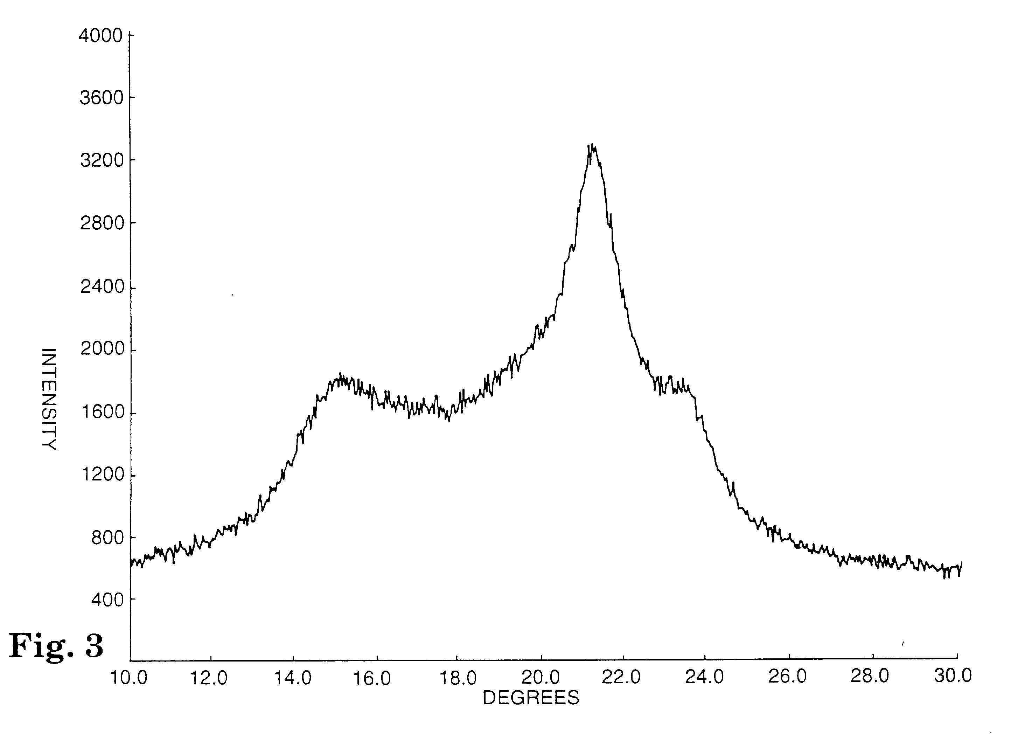

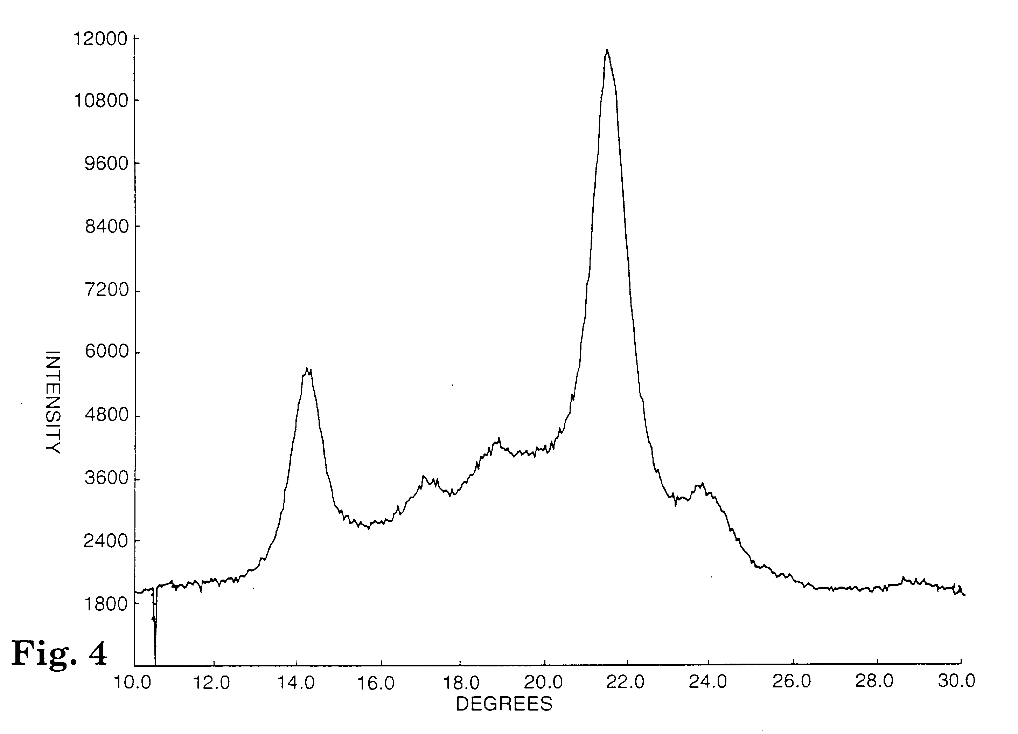

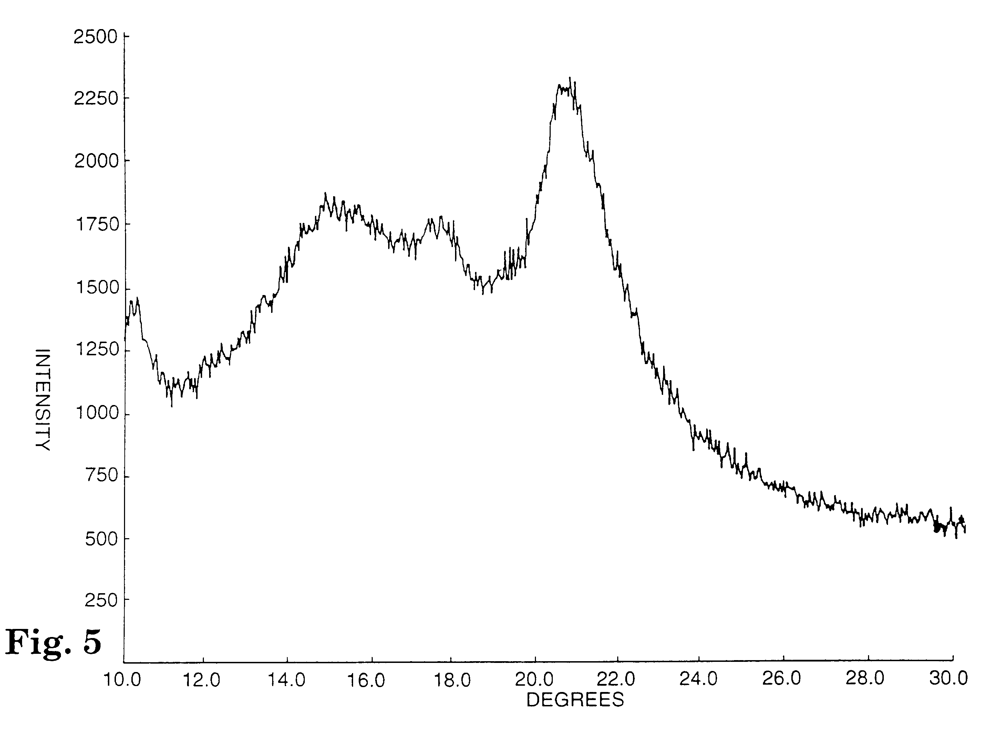

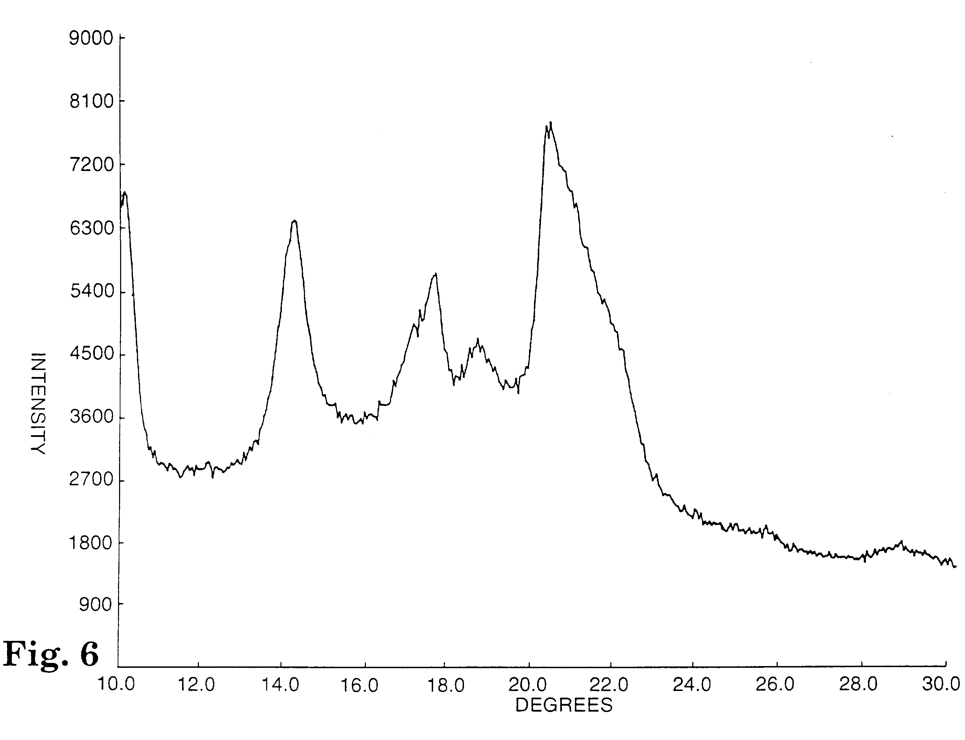

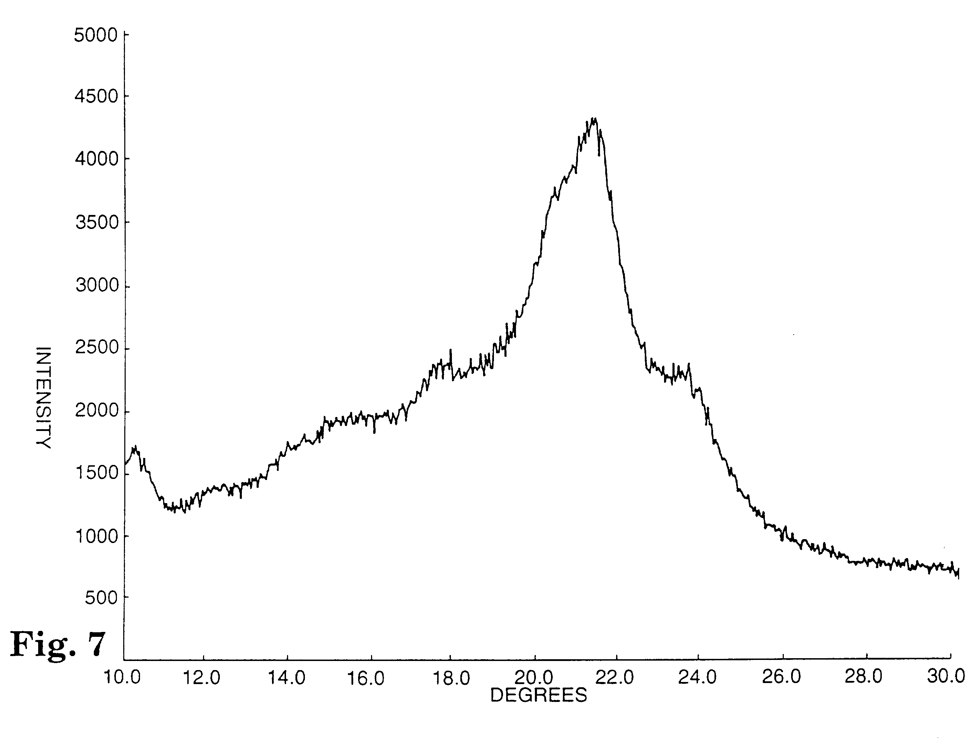

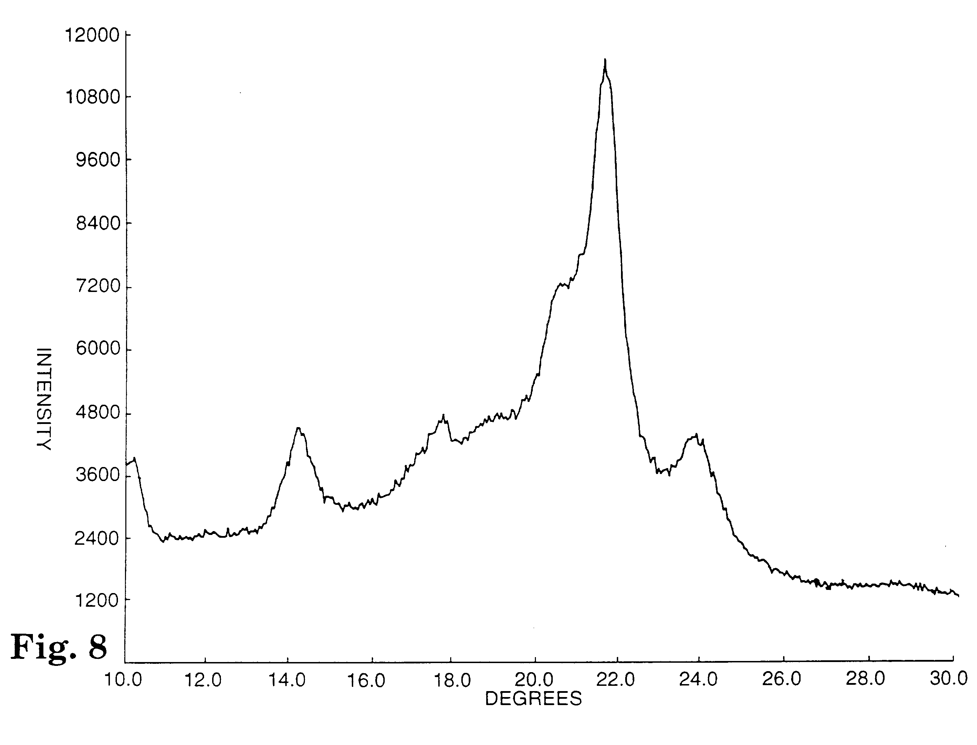

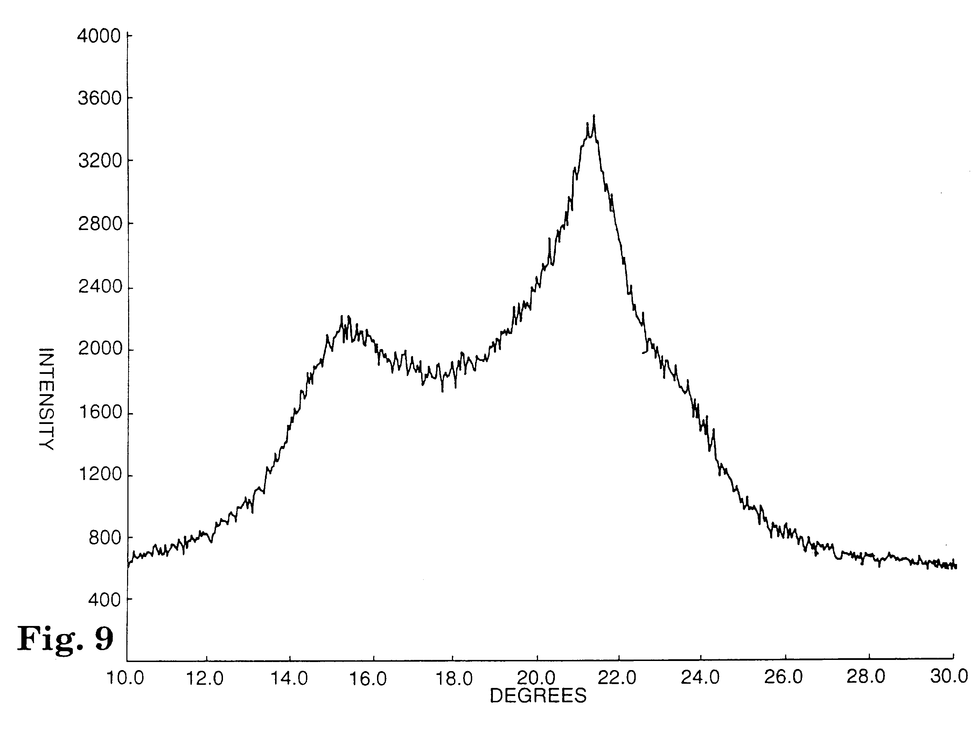

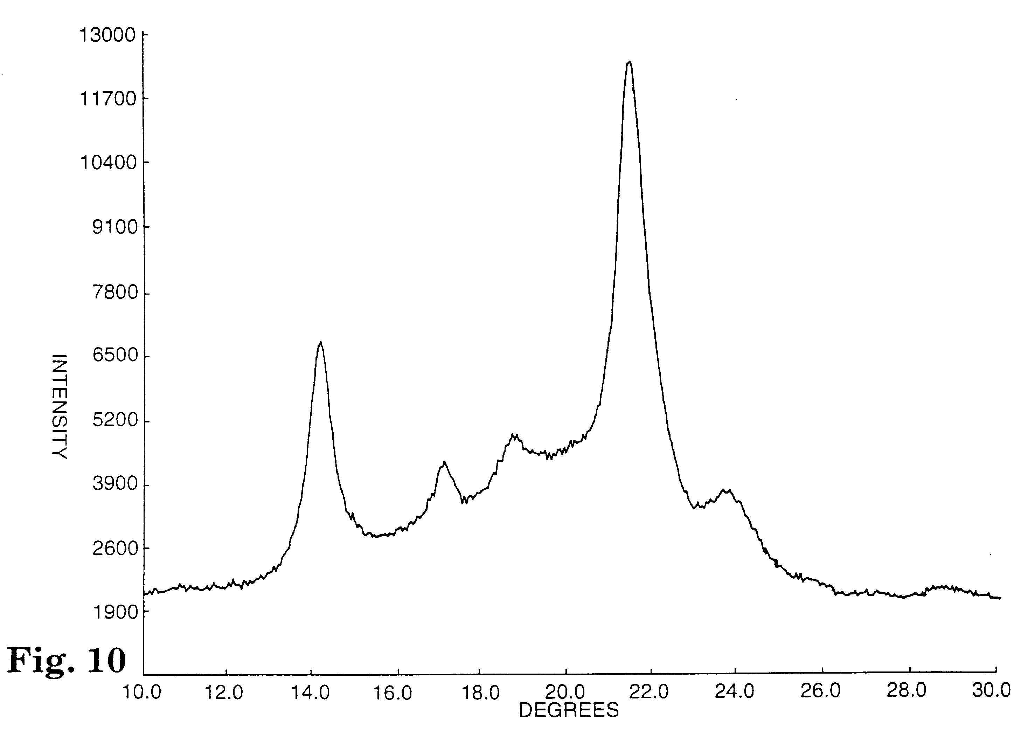

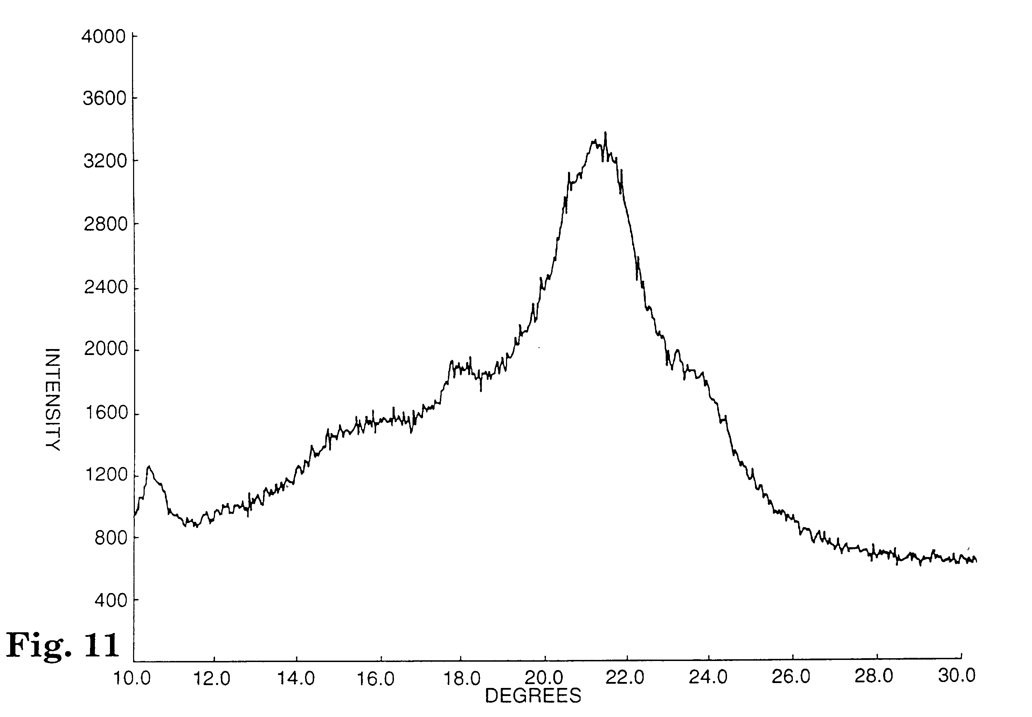

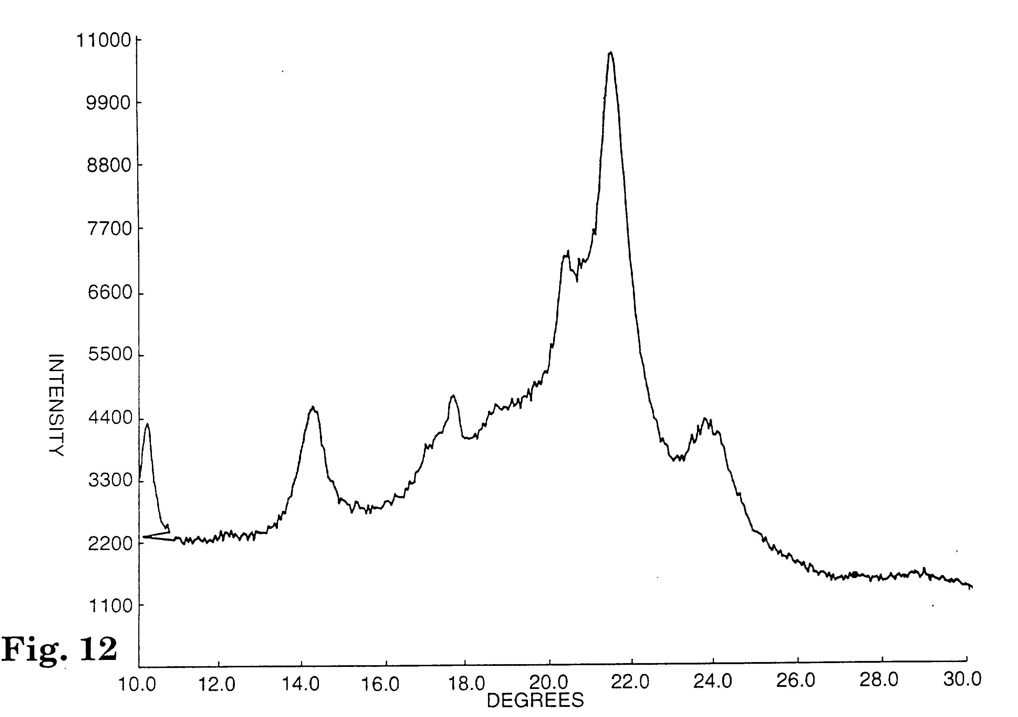

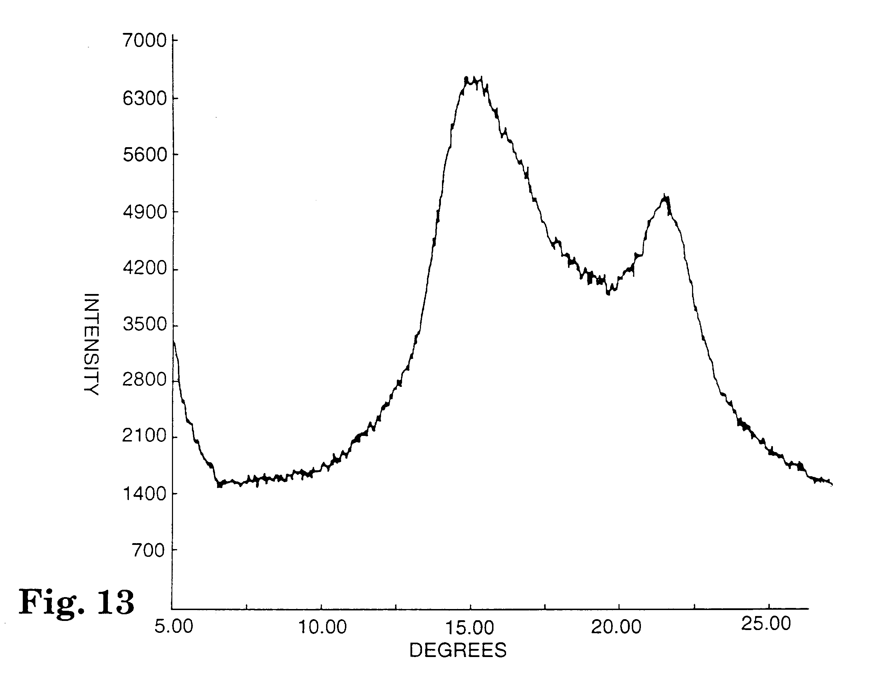

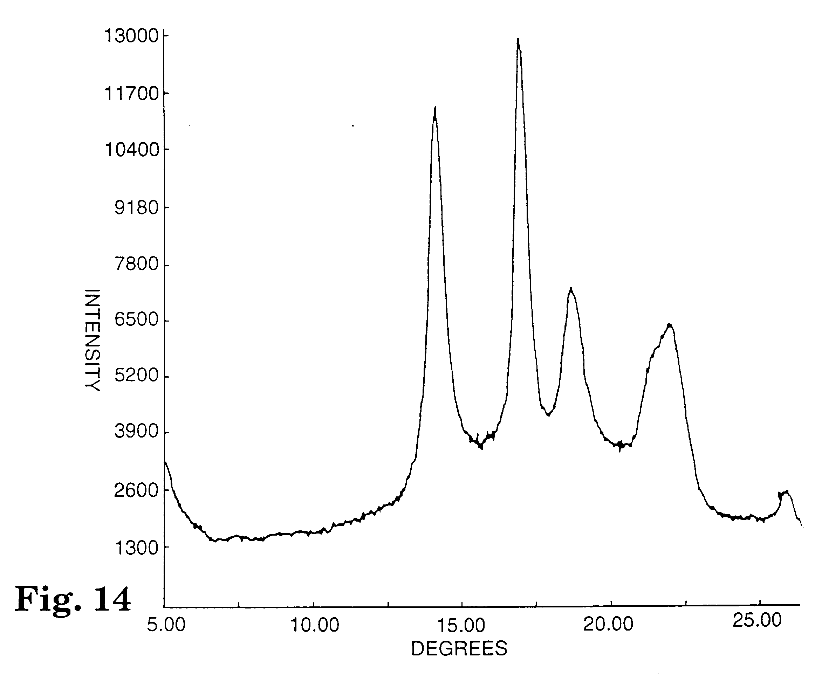

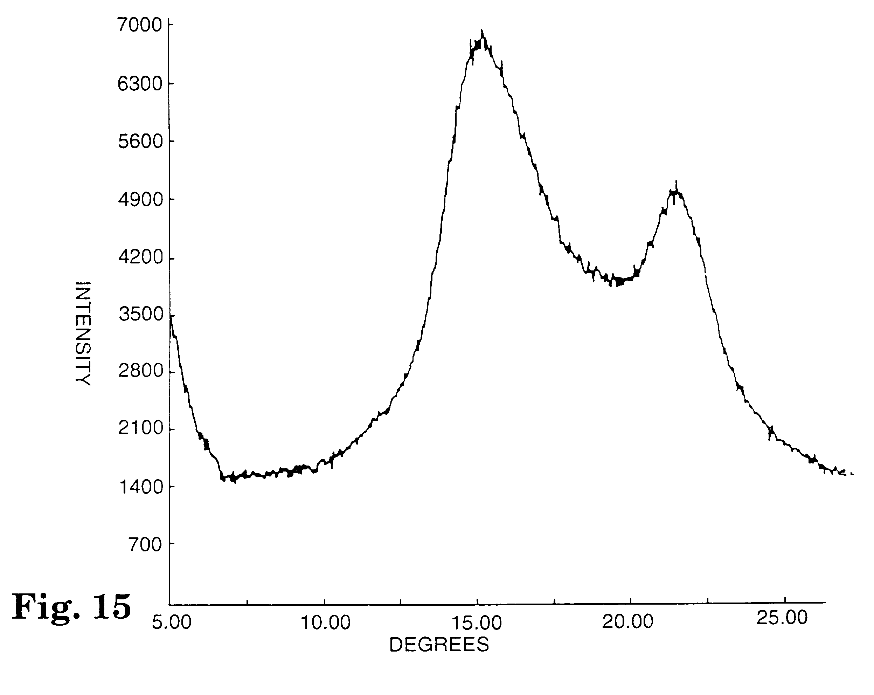

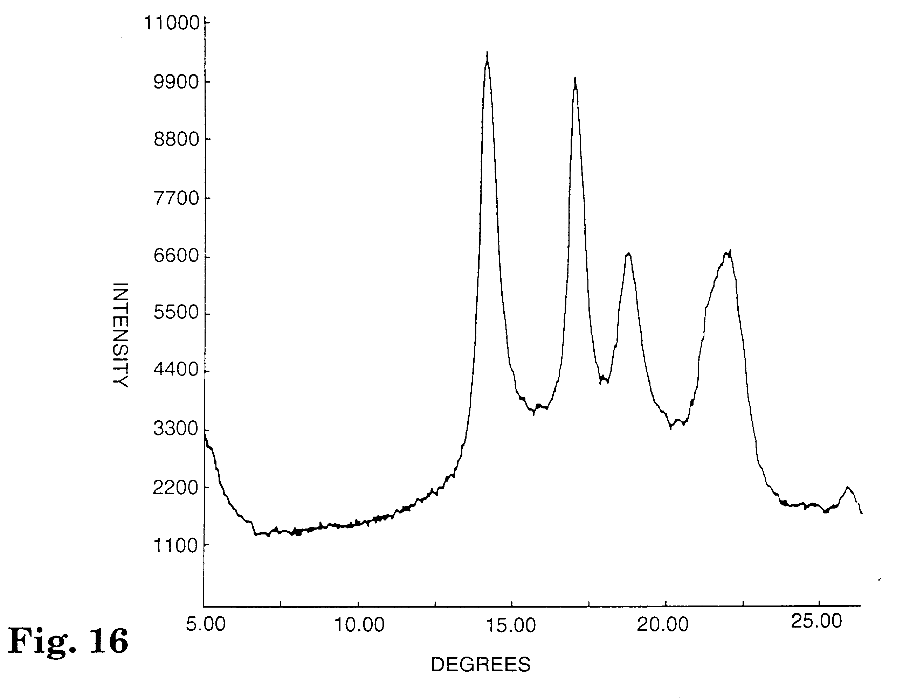

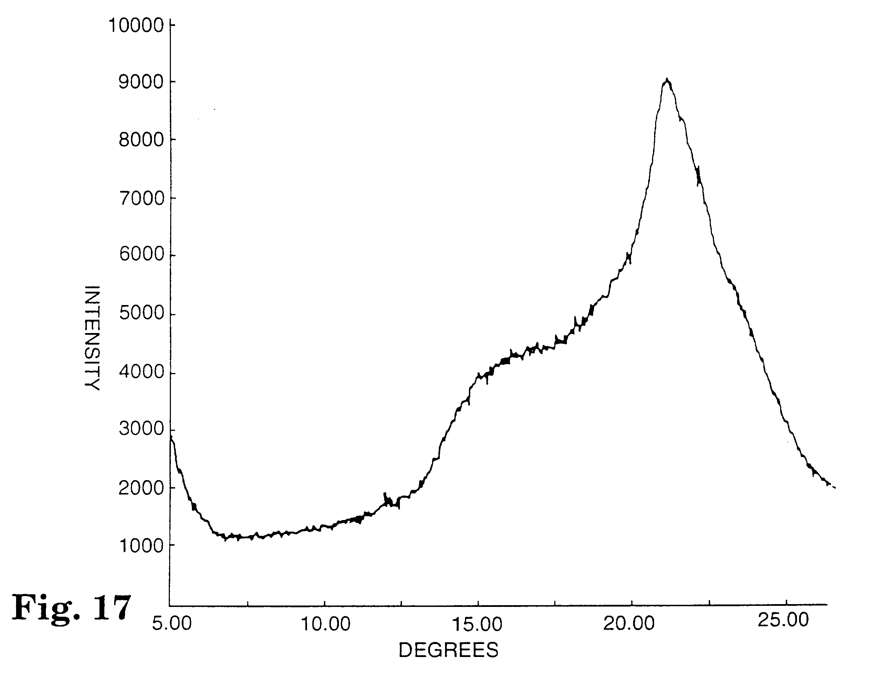

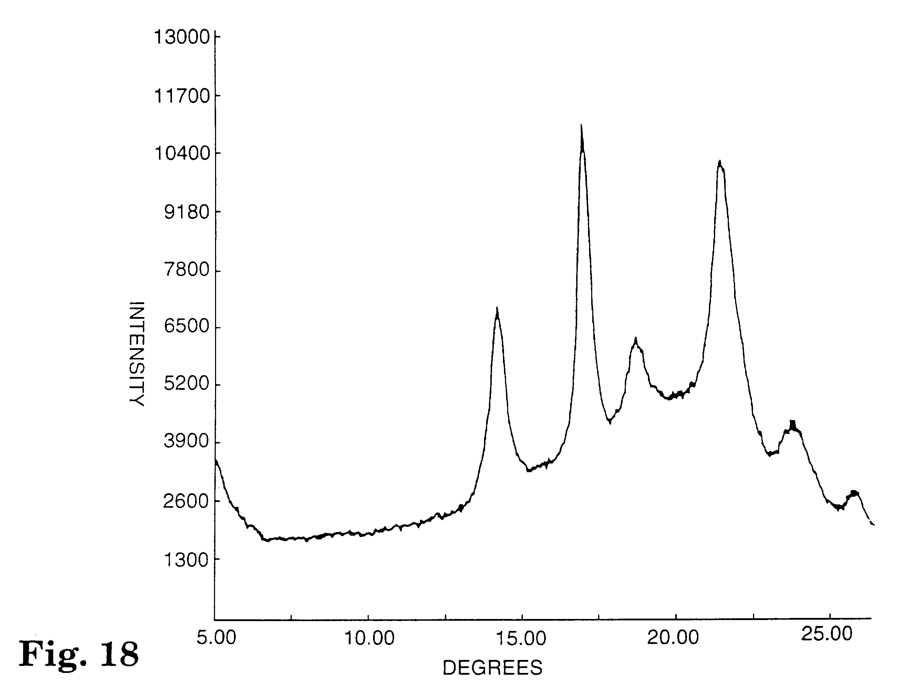

Crystalline or mesophase structures of the single layer films were determined by wide-angle x-ray diffraction (WAXD), and compared to the WAXD of mesomorphous polypropylene homopolymer illustrated in FIG. 1 (Example 1), and of crystalline polypropylene homopolymer illustrated in FIG. 2 (Comparative Example 18). Representative examples of these WAXD patterns are shown in FIGS. 1-18. No WAXD was determined for Example 5/Comparative Example 22, since the films comprised pure EVA copolymer. Throughout the Figures, the mesophase form of the quenched mesopolymer blends of the present invention is readily distinguishable from the crystalline form of the nonquenched polymer blend films. For example, the quenched mesopolymer blend film of PP and EVA of Example 3, illustrated in FIG. 3, has a mesophase form comparable to mesomorphous polypropylene homopolymer (i.e. FIG. 1), while the nonquenched crystalline polymer blend film of Comparative Example 20, illustrated in FIG. 4, exhibits an isotactic I crystalline form comparable to that of crystalline polypropylene homopolymer (i.e. FIG. 2). Similarly, the quenched mesopolymer blend film of PP:PB:EVA of Example 8, illustrated in FIG. 7, clearly displays a mesophase form, while the comparable nonquenched blend film of Comparative Example 25, illustrated in FIG. 8, shows a crystalline form analogous to that of pure crystalline polypropylene homopolymer (FIG. 2). Furthermore, the mesophase form of the quenched mesopolymer blend films is maintained even when the blend components comprise mesocopolymers (i.e. FIGS. 13, 15 and 17), and are in sharp contrast to the crystalline form displayed by the comparative copolymer blend films in the nonquenched state (i.e. FIGS. 14, 16 and 18).

Tensile properties were performed on the Example and Comparative Example films using an Instron® model 1122 machine, using 5 cm x 2.5 cm film samples. Each sample was deformed at a strain rate of one thousand percent (1000%) per minute (sample gauge length of 5 cm and a crosshead speed of 50 cm per minute) using ASTM D882-88 procedures. In each case, at least three samples were measured for each value reported. Table 2 lists modulus values for each of the Example and Comparative Example films. The modulus (Young's modulus, as reported in Mega Pascals (MPa)) for the quenched mesopolymer blend films of Examples 1-17 is lower than for Comparative Example films 18-34, that are formed from the comparable crystalline polymer blends. Thus, the mesopolymer blends of the present invention provide softer structures than comparable crystalline form materials.

**Table 2**

| Modulus values (MPa) for Example films 1-17, and Comparative Example films 18-34. | | | |
|---|---|---|---|
| Example No./ Comparative Example No. | Polymer Blend Composition | Casting Roll Temperature (°C) | Modulus (MPa) |
| 1/18 | PP1:EVA | 10/66 | 352/431 |
| 2/19 | PP1:EVA | 10/66 | 275/315 |
| 3/20 | PP1:EVA | 10/66 | 189/223 |
| 4/21 | PP1:EVA | 10/66 | 109/132 |
| 5/22 | PP1:EVA | 10/66 | 23/42 |
| 6/23 | PP1:PB:EVA | 10/66 | 147/186 |
| 7/24 | PP1:PB:EVA | 10/66 | 115/161 |
| 8/25 | PP1:PB:EVA | 10/66 | 93/118 |
| 9/26 | PP1:PB:EVA | 10/66 | 72/73 |
| 10/27 | PP1:EAA | 10/66 | 259/346 |
| 11/28 | PP1:EAA | 10/66 | 203/238 |
| 12/29 | PP1:EAA | 10/66 | 124/137 |
| 13/30 | PP1:PB:EAA | 10/66 | 125/181 |
| 14/31 | PP1:PB:EAA | 10/66 | 113/130 |
| 15/32 | CO1:CO2 | 18/66 | 248/380 |
| 16/33 | PP2:CO1 | 18/66 | 306/408 |
| 17/34 | EVA:CO1 | 18/66 | 136/187 |

The mesopolymer blend films of Examples 2, 3, 7, 10, 11, and 13, and crystalline polymer blend films of Comparative Examples 19, 20, 24, 27, 28 and 30 were electron beam irradiated at a dosage of 50 kGy (5 Mrads) and then immediately placed in liquid nitrogen. Electron paramagnetic resonance (EPR) analysis was performed by first warming the films to room temperature, cutting them to 1.3 cm x 7.6 cm in size, weighing, and then mounting the film strips in tubes. This technique allows reproducible sample positioning in the EPR cavity. Radical peak heights were recorded for each sample as a function of elapsed time from the initial measurement using a Varian® model 4502 spectrometer with a 23 cm magnet operating in the "X"-band. Fremy's salt was used as the magnetic field reference. Peak height represents radical concentration as measured in spins/gram. Initial runs were used to estimate radical concentration, and the declining numbers are proportional to the initial number. Since different instrument settings were used for some samples, all numbers were normalized. Spin concentration was calibrated against the National Bureau of Standards No. 261 Ruby Standard.

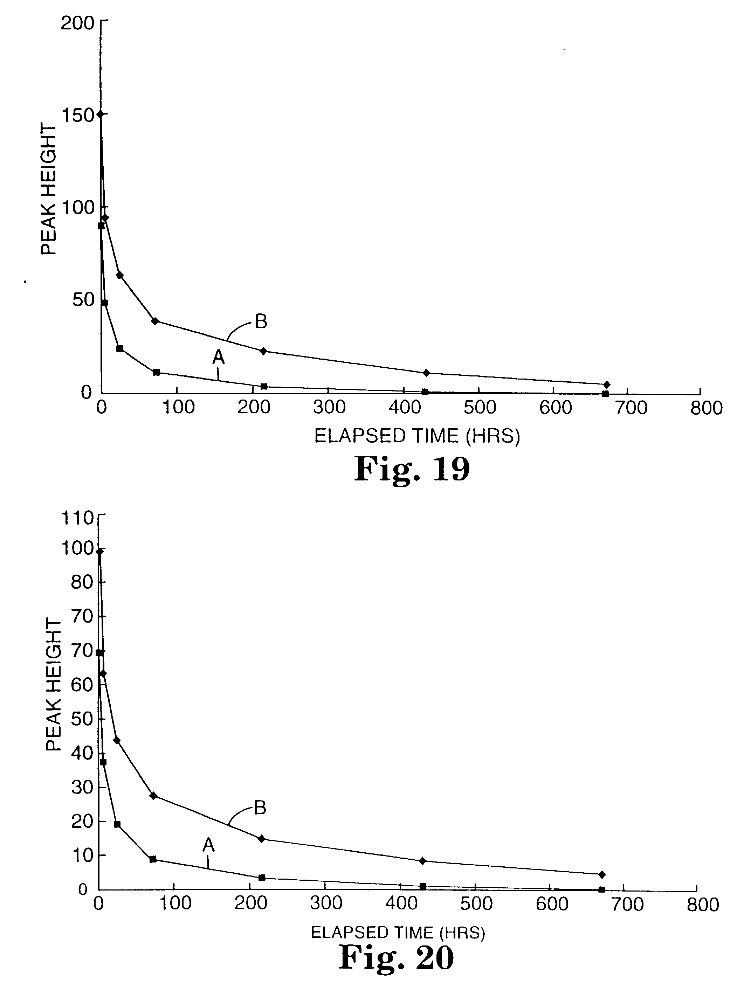

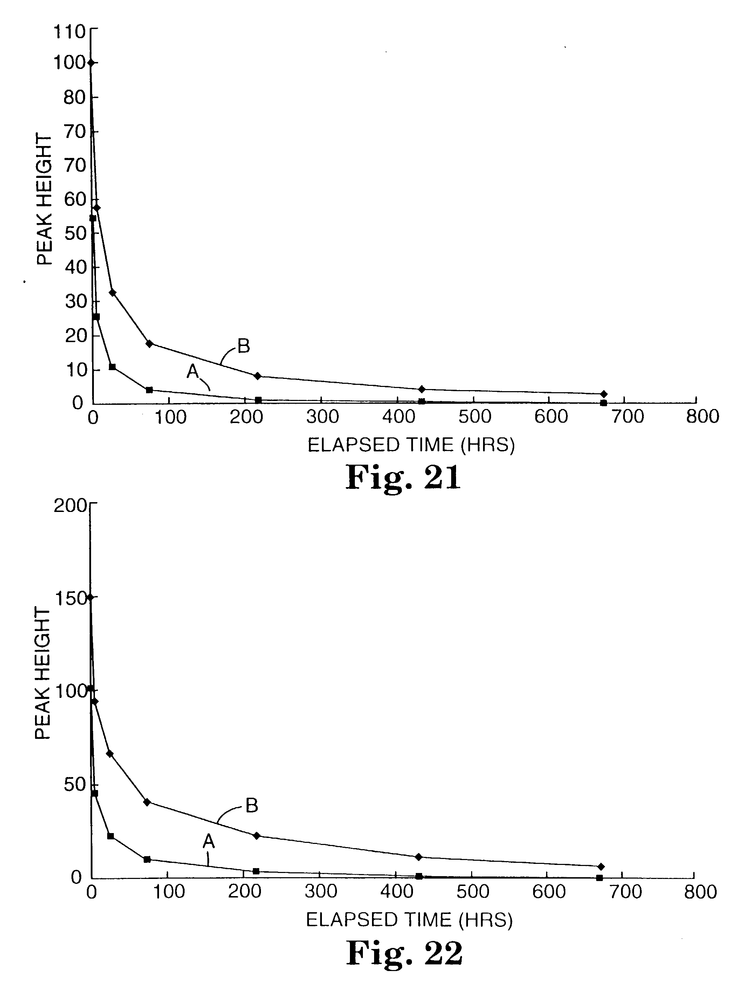

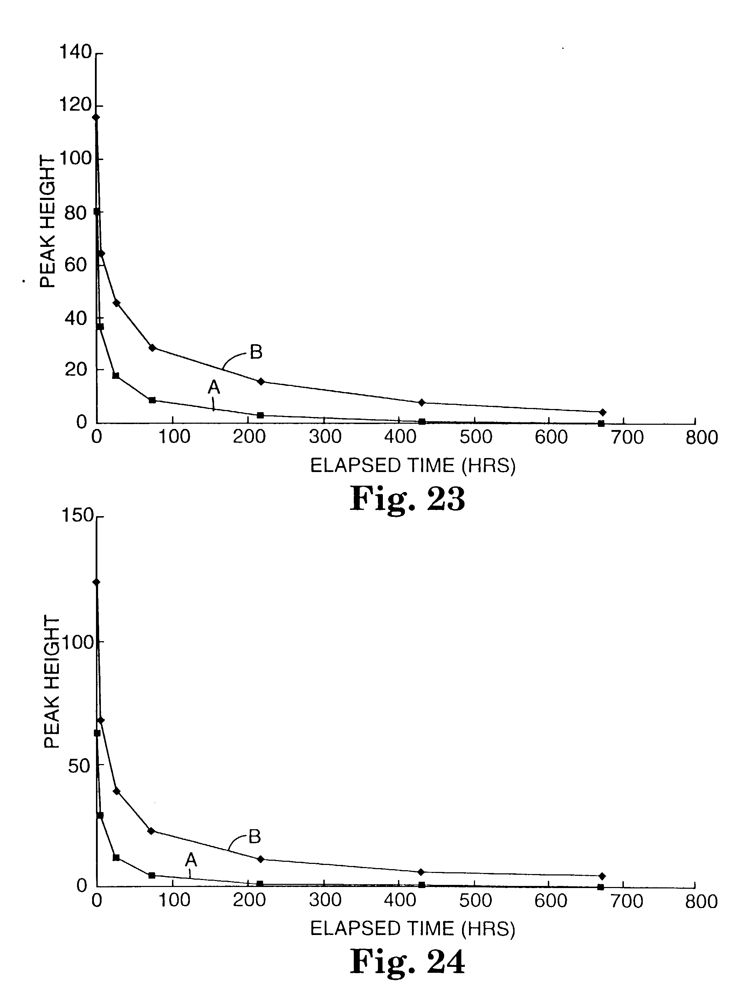

Normalized radical peak height in spins/gram as a function of elapsed time (hours) for the copolymer films is shown in FIGS. 19 through 24. In all cases, radial decay occurs in the quenched films (e.g. Examples 2, 7 and 10) at a much faster rate than in the comparison nonquenched films (e.g. Comparison Examples 19, 24 and 27). For example, as FIG. 19 illustrates, radical decay occurs at a consistently faster rate for the quenched mesopolymer blend film of PP:EVA of Example 2 (line A) in comparison to the nonquenched crystalline film of Comparison Example 19 (line B). Likewise, analogous results are shown with the quenched mesopolymer blend film of PP:EAA of Example 10 (line A) and the nonquenched crystalline polymer blend film of Comparison Example 27 (line B) in FIG. 22. Thus, the quenched films comprising the mesopolymer blends are expected to maintain their structural integrity and properties to a greater extent than their nonquenched crystalline polymer blend counterparts, since the radicals available for degradation are reduced more rapidly in the quenched films than in the nonquenched films.

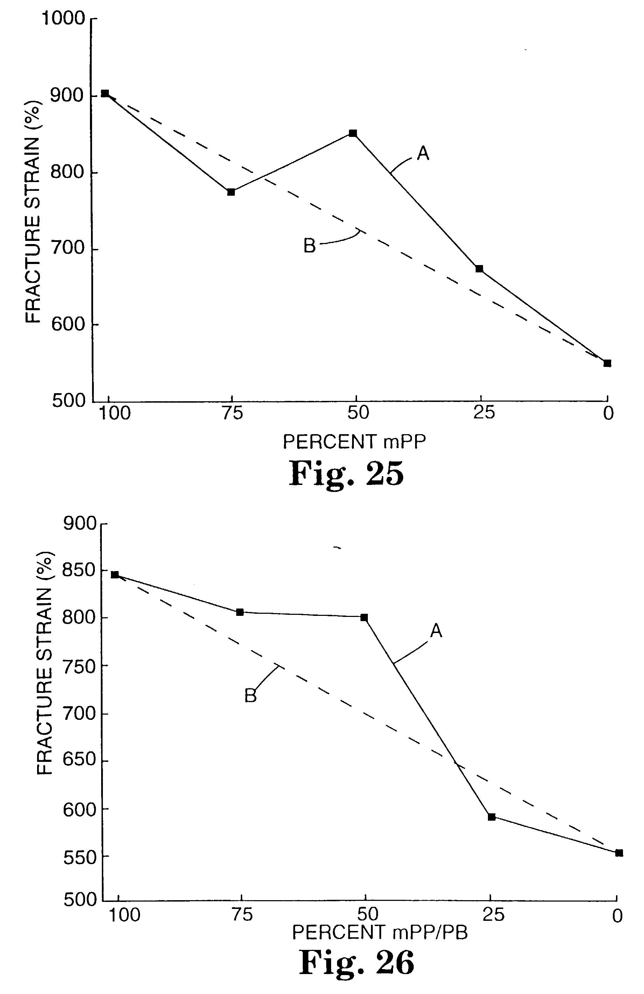

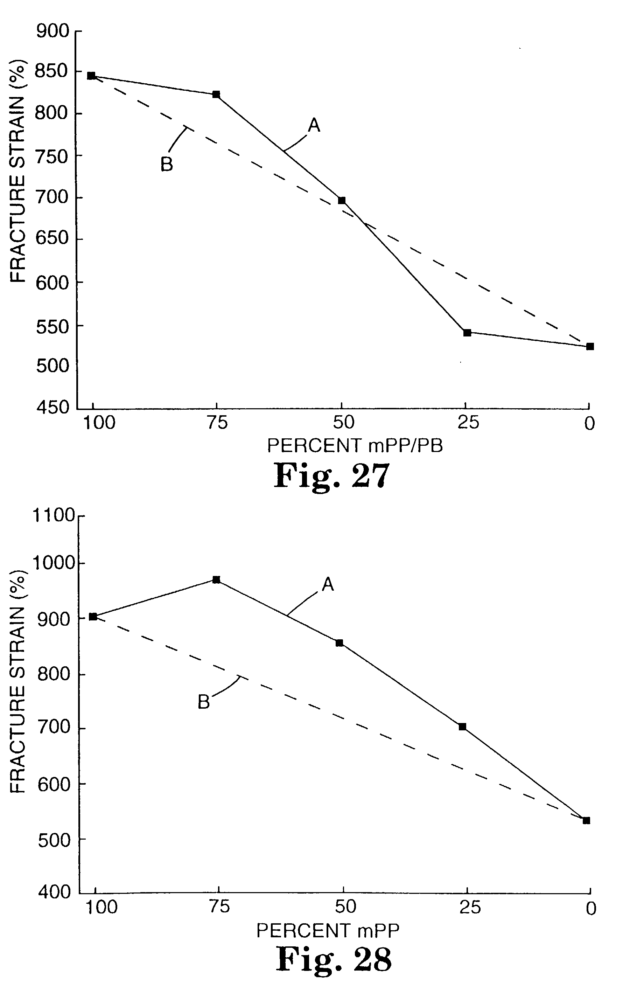

The effect of the amount of mesomorphous polypropylene (mPP) in the mesopolymer blends on the fracture strain (percent elongation to break) of various Example films is shown in FIGS. 25 through 28. In particular, each of these Figures shows synergy in fracture strain at various concentrations that is greater than would be expected under the Rule of Mixtures, as that term is defined herein. For example, as can be seen in FIG. 25, the mesopolymer blends of mesomorphous polypropylene (mPP) with ethylene vinyl acetate copolymer, where mPP comprises approximately seventy percent (70%) or less of the mesopolymer blend, exhibit greater fracture strain strength (line A), than would be expected from such blends under the Rule of Mixtures (line B), as that term is defined herein. Likewise, as FIG. 28 illustrates, all tested concentrations of the mesopolymer blends of mPP and ethylene acrylic acid copolymer showed greater fracture strain strength (line A) than would be expected of such polymer blends under the Rule of Mixtures (line B). Thus, using polymer blends of mPP with EVA, EAA and/or polybutylene (PB) results in films which are surprisingly tougher (i.e. display a greater strain to fracture) than would be expected.

### Comparative Examples 37-40 and Examples 41-44

Samples of the mesopolymer blend films of Examples 3 and 11 were coated with acrylic acid (AA) monomer (Examples 41 and 43) or dimethylacrylamide (DMA) monomer (Examples 42 and 44). These coated films were then irradiated using an electron beam at a dosage of 50 kGy (5 Mrads) in an inert nitrogen atmosphere, resulting in the grafting of these monomers to the surfaces of the coated films. In addition, two control films each were produced from the Example 3 and Example 5 films. The first control films comprised uncoated and nonradiated samples of the Example 3 and Example 11 films (Comparative Examples 37 and 39). The second control films comprised uncoated films irradiated at a dosage of 50 kGy (Comparative Examples 38 and 40) according to the same procedure as for Examples 41-44.

To assess the strength of the grafted layer, 180° peel adhesion measurements were performed according to the following procedure. A 2.5 cm wide, 20.3 cm long strip of pressure-sensitive adhesive tape (Scotch™ brand tape no. 8411; 3M Company) was adhered to a 10.1 cm wide, 15.2 cm long sheet of each of the Example and Comparative Example films, with a free end of the tape extending beyond the end of each film. The sample films were then rolled twice with a 1.35 kg hard rubber roller to ensure contact between the adhesive and the sample films. The samples were then aged at room temperature (22°C) for 24 hours, after which the free end of the tape was removed from the samples at a rate of 15.2 cm/min using a slip/peel testing machine (Instrumentors, Inc., Strongsville, OH). The graft monomers employed, electron beam radiation dosage employed, and the peak peel adhesion force measured in grams/2.5 cm for the sample films of Comparative Examples 37-40, and Examples 41-44 are shown in Table 3.

**Table 3**

| Graft monomers used, electron beam radiation dosage employed, and peak peel adhesion measured in grams/2.5 cm for the sample films of Comparative Examples 37-41, and Examples 41-44. | | | |
|---|---|---|---|
| Example Number | Graft Monomer | E-beam Dose (kGy) | Peak Peel Force (g/2.5 cm) |
| Comp. Ex. 37 | none | 0 | 4.4 |
| Comp. Ex. 38 | none | 50 | 7.3 |
| Ex. 41 | AA | 50 | 13.7 |
| Ex. 42 | DMA | 50 | 13.7 |
| Comp. Ex. 39 | none | 0 | 5.4 |
| Comp. Ex. 40 | none | 50 | 8.4 |
| Ex. 43 | AA | 50 | 13.2 |
| Ex. 44 | DMA | 50 | 13.1 |

The data of Table 3 show that mesopolymer blend films of the present invention with an additional monomer layer grafted thereto have higher peel strength than the same blend films lacking in the additional monomer layer, and when not exposed to electron beam radiation in an inert atmosphere. Thus, adhesion promoting layers, as well as other layers, can be added to the mesopolymer blend films of the present invention utilizing the above techniques.

## Claims

1. A mesopolymer blend comprising an overall mesophase propylene-based material and at least one percent by weight of a blend copolymer selected from the group consisting of a mesocopolymer, an ethylene vinyl acetate copolymer, an ethylene acrylic acid copolymer, and combinations thereof, wherein said mesophase propylene-based material and said blend copolymer are different, and wherein said blend has been rapidly quenched from its melt state to provide a mesomorphic phase.

2. A mesopolymer blend according to claim 1, wherein the mesophase propylene-based material is selected from the group consisting of mesomorphous polypropylene, a mesocopolymer and combinations thereof.

3. A mesopolymer blend according to claim 1, wherein the mesocopolymer comprises a mesophase propylene-based material combined with at least one moiety.

4. A mesopolymer blend according to claim 3, wherein the moiety is selected from the group consisting of a graft-molecule, a monomer, a polymer, and combinations thereof.

5. A mesopolymer blend according to claim 4, wherein the monomer is selected from the group consisting of ethylene, butylene, pentene, methylpentene, and combinations thereof.

6. A mesopolymer blend according to any preceding claim, wherein the polymer blend further comprises polybutylene.

7. A mesopolymer blend according to any of preceding claims 1 to 6 wherein the blend copolymer comprises at least five percent by weight of the weight of the polymer blend.

8. A mesopolymer blend according to any one of preceding claims 1 to 6 wherein the blend copolymer comprises at least ten percent by weight of the weight of the polymer blend.

9. A mesopolymer blend according to any one of preceding claims 1 to 6 wherein the blend copolymer comprises from twenty percent to eighty percent by weight of the polymer blend.

10. A mesopolymer blend according to any one of preceding claims 1 to 6 wherein the blend copolymer comprises from twenty-five percent to seventy-five percent by weight of the polymer blend.

11. A mesopolymer blend according to any one of preceding claims 1 to 6 wherein the blend copolymer comprises from forty percent to sixty percent by weight of the polymer blend.

12. A mesopolymer blend according to any one of preceding claims 1 to 6 wherein the blend copolymer comprises greater than ninety percent by weight of the polymer blend.

13. A mesopolymer blend according to any one of preceding claims 1 to 6 wherein the blend copolymer comprises greater than ninety-five percent by weight of the polymer blend.

14. A mesopolymer blend according to any one of preceding claims 1 to 6 wherein the blend copolymer comprises greater than ninety-nine percent by weight of the polymer blend.

15. A mesopolymer blend according to any preceding claim wherein the blend exhibits a synergistic enhancement of at least one physical property above what would be expected under the Rule of Mixtures.

16. A mesopolymer blend according to claim 15 wherein the blend exhibits a synergistic increase in fracture strain.

17. A mesopolymer blend according to any preceding claim wherein the blend exhibits a modulus of elasticity that is less than for a comparable blend of a crystalline copolymer and a propylene-based material.

18. A mesopolymer blend according to any preceding claim wherein the blend is capable of maintaining its structural integrity after exposure to a dosage of ionizing radiation of from between 1 kGy to 200 kGy.

19. A mesopolymer blend according to any preceding claim wherein the blend is heat-sealable to itself or other heat-sealable materials.

20. A mesopolymer blend according to any preceding claim wherein the blend comprises a film, a fiber, a microfiber, a tube, or a pouch.

21. A mesopolymer blend according to any preceding claim wherein the blend further comprises a graft layer affixed to a surface of the polymer blend by a dose of ionizing radiation.

22. A mesopolymer blend according to claim 21, wherein the graft layer enhances one or more properties of the polymer blend, including surface adhesion, coefficient of friction, oxygen permeability, moisture permeability, or combinations thereof.

23. A mesopolymer blend according to claim 22, wherein the graft layer comprises a surface adhesion layer formed by affixing to the surface of the polymer blend a compound selected from the group consisting of acrylic acid, dimethylacrylamide, N-vinyl-2-pyrrolidone, a copolymer of N-vinyl-2-pyrrolidone and trimethylolpropanetriacrylate, glycidyl acrylate, hydroxyethyl acrylate, hydroxymethyl acrylate, 2-vinyl pyridine, sulfoethyl methacrylate, diisopropylacrylamide, N,N-diethylamino acrylate, and combinations thereof.

24. A method of preparing a mesopolymer blend, comprising:
(a) blending a propylene-based material and at least one percent by weight of a blend copolymer selected from the group consisting of a mesocopolymer, an ethylene vinyl acetate copolymer, an ethylene acrylic acid copolymer, and combinations thereof, and wherein said mesophase propylene-based material and said blend copolymer are different to form a polymer blend;
(b) extruding the polymer blend; and
(c) quenching the extruded polymer blend immediately after extrusion to provide a mesopolymer blend having an overall mesophase propylene-based material contained therein, wherein the quenching occurs at a temperature of less than 38 °C.

25. A method of preparing a mesopolymer blend according to claim 24, further comprising, mixing polybutylene with the blend copolymer and propylene-based material to provide the polymer blend.

26. A method of preparing a mesopolymer blend according to any one of preceding claims 24 to 25, further comprising, after the quenching step, irradiating the mesopolymer blend with a dosage of ionizing radiation of from between 1 kGy to 200 kGy.

27. A method of preparing a mesopolymer blend according to any one of preceding claims 24 to 26, further comprising, after the quenching step, grafting a graft layer to a surface of the mesopolymer blend through exposure to a dose of ionizing radiation.

28. A polymer blend according to claim 27, wherein the ionizing radiation comprises electron beam radiation at from 5 kGy to 200 kGy.

29. A method of preparing a mesopolymer blend according to any one of preceding claims 27 to 28, wherein the graft layer enhances one or more properties of the mesopolymer blend, including surface adhesion, coefficient of friction, oxygen permeability, moisture permeability, or combinations thereof.

30. An article formed from a mesopolymer blend comprising an overall mesophase propylene-based material and at least one percent by weight of a blend copolymer selected from the group consisting of a mesocopolymer, an ethylene vinyl acetate copolymer, an ethylene acrylic acid copolymer, and combinations thereof, and wherein said mesophase propylene-based material and said blend copolymer are different and wherein said blend has been rapidly quenched from its melt state to provide a mesomorphic phase.

31. An article according to claim 30, wherein the article comprises a film, a fiber, a microfiber, a tube or a pouch.

32. An article according to any one of preceding claims 30 to 31 further comprising a graft layer affixed to a surface of the article by a dose of ionizing radiation.

33. An article according to claim 32, wherein the graft layer enhances one or more properties of the article, including surface adhesion, coefficient of friction, oxygen permeability, moisture permeability, or combinations thereof.

34. An article according to claim 33, wherein the graft layer comprises a surface adhesion layer formed by affixing to the article a compound selected from the group consisting of acrylic acid, dimethylacrylamide, N-vinyl-2-pyrrolidone, a copolymer of N-vinyl-2-pyrrolidone and trimethylolpropanetriacrylate, glycidyl acrylate, hydroxyethyl acrylate, hydroxymethyl acrylate, 2-vinyl pyridine, sulfoethyl methacrylate, diisopropylacrylamide, N,N-diethylamino acrylate, and combinations thereof.

35. Use of an article formed from a mesopolymer blend comprising:
(a) providing an article according to claim 30; and
(b) interposing the article between a protected environment and an external environment such that the protected environment remains substantially free from contamination.

36. Use according to claim 35, wherein the article comprises a packaging film containing a degradable product in the protected environment.

37. Use according to claim 36, wherein the degradable product comprises a food product or a pharmaceutical product.

38. Use according to claim 35, wherein the article comprises a transdermal drug delivery patch, a medical tape, medical tubing, or an ostomy pouch.

39. Use according to claim 35, wherein the article further comprises a graft layer affixed to a surface of the article by a dose of ionizing radiation.

## Patentansprüche

1. Mesopolymerblend, umfassend ein Material auf Propylen-Basis mit Gesamt-Mesophase und mindestens ein Gewichtsprozent eines Blendcopolymers, ausgewählt aus der Gruppe, bestehend aus einem Mesocopolymer, einem Ethylen/Vinylacetat-Copolymer, einem Ethylen/Acrylsäure-Copolymer sowie Kombinationen davon, wobei das Material auf Propylen-Basis mit Mesophase und das Blendcopolymer voneinander verschieden sind und wobei das Blend aus seinem schmelzflüssigen Zustand rasch gequencht wurde, um eine mesomorphe Phase zu schaffen.

2. Mesopolymerblend nach Anspruch 1, bei welchem das Material auf Propylen-Basis mit Mesophase ausgewählt wird aus der Gruppe, bestehend aus mesomorphem Polypropylen, einem Mesocopolymer sowie Kombinationen davon.

3. Mesopolymerblend nach Anspruch 1, bei welchem das Mesocopolymer ein Material auf Propylen-Basis mit Mesophase aufweist, das mit mindestens einem Teil kombiniert ist.

4. Mesopolymerblend nach Anspruch 3, bei welchem der Teil ausgewählt wird, bestehend aus einem aufpolymerisierten Molekül, einem Monomer, einem Polymer sowie Kombinationen davon.

5. Mesopolymerblend nach Anspruch 4, bei welchem das Monomer ausgewählt wird aus der Gruppe, bestehend aus Ethylen, Butylen, Penten, Methylpenten sowie Kombinationen davon.

6. Mesopolymerblend nach einem der vorgenannten Ansprüche, bei welchem das Polymerblend ferner Polybutylen umfaßt.

7. Mesopolymerblend nach einem der vorgenannten Ansprüche 1 bis 6, bei welchem das Blendcopolymer mindestens fünf Gewichtsprozent der Masse des Polymerblends ausmacht.

8. Mesopolymerblend nach einem der vorgenannten Ansprüche 1 bis 6, bei welchem das Blendcopolymer mindestens zehn Gewichtsprozent der Masse des Polymerblends ausmacht.

9. Mesopolymerblend nach einem der vorgenannten Ansprüche 1 bis 6, bei welchem das Blendcopolymer zwanzig Prozent bis achtzig Gewichtsprozent des Polymerblends ausmacht.

10. Mesopolymerblend nach einem der vorgenannten Ansprüche 1 bis 6, bei welchem das Blendcopolymer fünfundzwanzig Prozent bis fünfundsiebzig Gewichtsprozent des Polymerblends ausmacht.

11. Mesopolymerblend nach einem der vorgenannten Ansprüche 1 bis 6, bei welchem das Blendcopolymer vierzig Prozent bis sechzig Gewichtsprozent des Polymerblends ausmacht.

12. Mesopolymerblend nach einem der vorgenannten Ansprüche 1 bis 6, bei welchem das Blendcopolymer mehr als neunzig Gewichtsprozent des Polymerblends ausmacht.

13. Mesopolymerblend nach einem der vorgenannten Ansprüche 1 bis 6, bei welchem das Blendcopolymer mehr als fünfundneunzig Gewichtsprozent des Polymerblends ausmacht.

14. Mesopolymerblend nach einem der vorgenannten Ansprüche 1 bis 6, bei welchem das Blendcopolymer mehr als neunundneunzig Gewichtsprozent des Polymerblends ausmacht.

15. Mesopolymerblend nach einem der vorgenannten Ansprüche, bei welchem das Blend eine synergistische Verstärkung mindestens einer der physikalischen Eigenschaften über das hinaus zeigt, was nach der Mischungsregel zu erwarten wäre.

16. Mesopolymerblend nach Anspruch 15, bei welchem das Blend eine synergistischer Erhöhung der Reißdehnung zeigt.

17. Mesopolymerblend nach einem der vorgenannten Ansprüche, bei welchem das Blend einen Elastizitätsmodul zeigt, der kleiner ist als für ein vergleichbares Blend aus einem kristallinen Copolymer und einem Material auf Propylen-Basis.

18. Mesopolymerblend nach einem der vorgenannten Ansprüche, bei welchem das Blend in der Lage ist, nach Exponierung an einer Dosis ionisierender Strahlung zwischen 1 kGy bis 200 kGy seinen strukturellen Zusammenhalt zu bewahren.

19. Mesopolymerblend nach einem der vorgenannten Ansprüche, bei welchem das Blend mit sich selbst oder anderen heißsiegelfähigen Materialien heißsiegelfähig ist.

20. Mesopolymerblend nach einem der vorgenannten Ansprüche, bei welchem das Blend eine Folie, eine Faser, eine Mikrofaser, einen Schlauch oder einen Beutel umfaßt.

21. Mesopolymerblend nach einem der vorgenannten Ansprüche, bei welchem das Blend ferner eine aufpolymerisierte Schicht umfaßt, die durch eine Dosis ionisierender Strahlung auf einer Oberfläche des Polymerblends fixiert ist.

22. Mesopolymerblend nach Anspruch 21, bei welchem die aufpolymerisierte Schicht eine oder mehrere Eigenschaften des Polymerblends verstärkt, einschließend Oberflächenhaftung, Reibungskoeffizient, Sauerstoff-Durchlässigkeit, Feuchtigkeitsdurchlässigkeit oder Kombinationen davon.

23. Mesopolymerblend nach Anspruch 22, bei welchem die aufpolymerisierte Schicht eine Oberflächenhaftschicht umfaßt, die erzeugt wird, indem auf der Oberfläche des Polymerblends eine Verbindung fixiert wird, die ausgewählt wird aus der Gruppe, bestehend aus: Acrylsäure, Dimethylacrylamid, N-Vinyl-2-pyrrolidon, einem Copolymer von N-Vinyl-2-pyrrolidon und Trimethylolpropantriacrylat, Glycidylacrylat, Hydroxyethylacrylat, Hydroxymethylacrylat, 2-Vinylpyridin, Sulfoethylmethacrylat, Diisopropylacrylamid, N,N-Diethylaminoacrylat sowie Kombinationen davon.

24. Verfahren zum Herstellen eines Mesopolymerblends, umfassend:
(a) Compoundieren eines Materials auf Propylen-Basis und mindestens ein Gewichtsprozent eines Blendcopolymers, ausgewählt aus der Gruppe, bestehend aus einem Mesocopolymer, einem Ethylen/Vinylacetat-Copolymer, einem Ethylen/Acrylsäure-Copolymer sowie Kombinationen davon, wobei das Material auf Propylen-Basis mit Mesophase und das Blendcopolymer voneinander verschieden sind, um ein Polymerblend zu bilden;
(b) Extrudieren des Polymerblends; sowie
(c) Quenchen des extrudierten Polymerblends unmittelbar nach Extrusion, um ein Mesopolymerblend zu schaffen, das ein darin Material auf Propylen-Basis mit Gesamt-Mesophase darin enthält, wobei das Quenchen bei einer Temperatur unterhalb von 38 °C erfolgt.

25. Verfahren zum Herstellen eines Mesopolymerblends nach Anspruch 24, ferner umfassend das Mischen von Polybutylen mit dem Blendcopolymer und dem Material auf Propylen-Basis, um das Polymerblend zu schaffen.

26. Verfahren zum Herstellen eines Mesopolymerblends nach einem der vorgenannten Ansprüche 24 und 25, welches nach dem Schritt des Quenchens ferner das Bestrahlen des Mesopolymerblends mit einer Dosis ionisierender Strahlung zwischen 1 kGy bis 200 kGy umfaßt.

27. Verfahren zum Herstellen eines Mesopolymerblends nach einem vorgenannten Ansprüche 24 bis 26, welches Verfahren ferner nach dem Schritt des Quenchens das Aufpolymerisieren einer Pfropfschicht auf eine Oberfläche des Mesopolymerblends durch Exponieren an einer Dosis ionisierender Strahlung umfaßt.

28. Polymerblend nach Anspruch 27, bei welchem die ionisierende Strahlung Elektronenstrahl-Strahlung von 5 kGy bis 200 kGy umfaßt.

29. Verfahren zum Herstellen eines Mesopolymerblends nach einem der vorgenannten Ansprüche 27 und 28, bei welchem die aufpolymerisierte Schicht eine oder mehrere Eigenschaften des Mesopolymerblends verstärkt, einschließend Oberflächenhaftung, Reibungskoeffizient, Sauerstoff-Durchlässigkeit, Feuchtigkeitsdurchlässigkeit oder Kombinationen davon.

30. Artikel, erzeugt aus einem Mesopolymerblend, umfassend ein Material aus Propylen-Basis mit Gesamt-Mesophase und mindestens ein Gewichtsprozent eines Blendcopolymers, ausgewählt aus der Gruppe, bestehend aus einem Mesocopolymer, einem Ethylen/Vinylacetat-Copolymer, einem Ethylen/Acrylsäure-Copolymer sowie Kombinationen davon, und bei welchem das Material auf Propylen-Basis mit Mesophase und das Blendcopolymer verschieden sind und bei welchem das Blend aus seinem schmelzflüssigen Zustand rasch gequencht wurde, um eine mesomorphe Phase zu schaffen.

31. Artikel nach Anspruch 30, wobei der Artikel eine Folie, eine Faser, eine Mikrofaser, einen Schlauch oder einen Beutel umfaßt.

32. Artikel nach einem der vorgenannten Ansprüche 30 und 31, ferner umfassend eine aufpolymerisierte Schicht, die an einer Oberfläche des Artikels durch eine Dosis ionisierender Strahlung fixiert ist.

33. Artikel nach Anspruch 32, bei welchem die aufpolymerisierte Schicht eine oder mehrere Eigenschaften des Artikels verstärkt, einschließend Oberflächenhaftung, Reibungskoeffizient, Sauerstoff-Durchlässigkeit, Feuchtigkeitsdurchlässigkeit oder Kombinationen davon.

34. Artikel nach Anspruch 33, bei welchem die aufpolymerisierte Schicht eine Oberflächenhaftschicht umfaßt, die erzeugt wird, indem an dem Artikel eine Verbindung fixiert wird, ausgewählt aus der Gruppe, bestehend aus: Acrylsäure, Dimethylacrylamid, N-Vinyl-2-pyrrolidon, einem Copolymer von N-Vinyl-2-pyrrolidon und Trimethylolpropantriacrylat, Glycidylacrylat, Hydroxyethylacrylat, Hydroxymethylacrylat, 2-Vinylpyridin, Sulfoethylmethacrylat, Diisopropylacrylamid, N,N-Diethylaminoacrylat sowie Kombinationen davon.

35. Verwendung eines Artikels, erzeugt aus einem Mesopolymerblend, umfassend:
(a) Bereitstellen eines Artikels nach Anspruch 30; und
(b) Anordnen des Artikels zwischen einer geschützten Umgebung und einer äußeren Umgebung derart, daß die geschützte Umgebung weitgehend frei von Kontamination frei bleibt.

36. Verwendung nach Anspruch 35, bei welchem der Artikel eine Verpackungsfolie umfaßt, enthaltend ein abbaubares Produkt in der geschützten Umgebung.

37. Verwendung nach Anspruch 36, wobei das abbaubare Produkt ein Lebensmittelprodukt oder ein pharmazeutisches Produkt umfaßt.

38. Verwendung nach Anspruch 35, wobei der Artikel ein Pflaster mit transdermaler verzögerter Freisetzung von Medikamenten, ein medizinisches Pflaster, medizinischen Schlauch oder ein Stomabeutel umfaßt.

39. Verwendung nach Anspruch 35, wobei der Artikel ferner eine aufpolymerisierte Schicht umfaßt, die an einer Oberfläche des Artikels durch eine Dosis ionisierender Strahlung fixiert ist.

## Revendications

1. Mélange mésopolymère comprenant un matériau à base de propylène globalement en phase mésomorphe et au moins un pour cent en poids d'un copolymère de mélange choisi dans le groupe comprenant un mésocopolymère, un copolymère d'éthylène et d'acétate de vinyle, un copolymère d'éthylène et d'acide acrylique, ainsi que des combinaisons de ces substances, dans lequel ledit matériau à base de propylène en phase mésomorphe et ledit copolymère de mélange sont différents, et dans lequel ledit mélange à l'état fondu a été refroidi rapidement afin d'obtenir une phase mésomorphe.

2. Mélange mésopolymère selon la revendication 1, dans lequel le matériau à base de propylène en phase mésomorphe est choisi dans le groupe comprenant le polypropylène mésomorphe, un mésocopolymère et des combinaisons de ces substances.

3. Mélange mésopolymère selon la revendication 1, dans lequel le mésocopolymère comprend un matériau à base de propylène en phase mésomorphe associé à au moins un fragment.

4. Mélange mésopolymère selon la revendication 3, dans lequel le fragment est choisi dans le groupe comprenant une molécule greffée, un monomère, un polymère, et des combinaisons de ceux-ci.

5. Mélange mésopolymère selon la revendication 4, dans lequel le monomère est choisi dans le groupe comprenant l'éthylène, le butylène, le pentène, le méthylpentène, et des combinaisons de ces substances.

6. Mélange mésopolymère selon l'une quelconque des revendications précédentes, dans lequel le mélange polymère comprend en outre du polybutylène.

7. Mélange mésopolymère selon l'une quelconque des revendications 1 à 6 précédentes, dans lequel le copolymère de mélange comprend au moins cinq pour cent en poids du poids du mélange polymère.

8. Mélange mésopolymère selon l'une quelconque des revendications 1 à 6 précédentes, dans lequel le copolymère de mélange représente au moins dix pour cent en poids du poids du mélange polymère.

9. Mélange mésopolymère selon l'une quelconque des revendications 1 à 6 précédentes, dans lequel le copolymère de mélange représente de vingt pour cent à quatre-vingts pour cent en poids du mélange polymère.

10. Mélange mésopolymère selon l'une quelconque des revendications 1 à 6 précédentes, dans lequel le copolymère de mélange représente de vingt-cinq pour cent à soixante-quinze pour cent en poids du mélange polymère.

11. Mélange mésopolymère selon l'une quelconque des revendications 1 à 6 précédentes, dans lequel le copolymère de mélange représente de quarante pour cent à soixante pour cent en poids du mélange polymère.

12. Mélange mésopolymère selon l'une quelconque des revendications 1 à 6 précédentes, dans lequel le copolymère de mélange représente plus de quatre-vingt-dix pour cent en poids du mélange polymère.

13. Mélange mésopolymère selon l'une quelconque des revendications 1 à 6 précédentes, dans lequel le copolymère de mélange représente plus de quatre-vingt-quinze pour cent en poids du mélange polymère.

14. Mélange mésopolymère selon l'une quelconque des revendications 1 à 6 précédentes, dans lequel le copolymère de mélange représente plus de quatre-vingt-dix-neuf pour cent en poids du mélange polymère.

15. Mélange mésopolymère selon l'une quelconque des revendications précédentes, dans lequel le mélange présente une amélioration par synergie d'au moins une propriété physique au-delà de ce que l'on pouvait espérer d'après la règle des mélanges.

16. Mélange mésopolymère selon la revendication 15, dans lequel le mélange présente une augmentation par synergie de la tension de rupture.

17. Mélange mésopolymère selon l'une quelconque des revendications précédentes, dans lequel le mélange présente un module d'élasticité qui est inférieur à celui d'un mélange comparable composé d'un copolymère cristallin et d'un matériau à base de propylène.

18. Mélange mésopolymère selon l'une quelconque des revendications précédentes, dans lequel le mélange est capable de conserver son intégrité structurelle après avoir été exposé à une dose de rayonnement ionisant comprise entre 1 kGy et 200 kGy.

19. Mélange mésopolymère selon l'une quelconque des revendications précédentes, dans lequel le mélange peut être thermosoudé sur lui-même ou sur d'autres matériaux thermosoudables.

20. Mélange mésopolymère selon l'une quelconque des revendications précédentes, dans lequel le mélange constitue un film, une fibre, une microfibre, un tube, ou une poche.

21. Mélange mésopolymère selon l'une quelconque des revendications précédentes, dans lequel le mélange comprend en outre une couche greffée fixée sur une surface du mélange polymère par une dose de rayonnement ionisant.

22. Mélange mésopolymère selon la revendication 21, dans lequel la couche greffée améliore une ou plusieurs propriétés du mélange polymère, notamment l'adhérence en surface, le coefficient de frottement, la perméabilité à l'oxygène, la perméabilité à l'humidité, ou des combinaisons de celles-ci.

23. Mélange mésopolymère selon la revendication 22, dans lequel la couche greffée comprend une couche d'adhérence en surface formée en fixant sur la surface du mélange polymère un composé choisi dans le groupe comprenant l'acide acrylique, le diméthylacrylamide, la N-vinyl-2-pyrrolidone, un copolymère de N-vinyl-2-pyrrolidone et de triacrylate de triméthylolpropane, l'acrylate de glycidyle, l'acrylate d'hydroxyéthyle, l'acrylate d'hydroxyméthyle, la 2-vinylpyridine, le méthacrylate de sulfoéthyle, le diisopropylacrylamide, le N,N-diéthylaminoacrylate, et des combinaisons de ces substances.

24. Procédé de préparation d'un mélange mésopolymère, consistant à :
(a) mélanger un matériau à base de propylène et au moins un pour cent en poids d'un copolymère de mélange choisi dans le groupe comprenant un mésocopolymère, un copolymère d'éthylène et d'acétate de vinyle, un copolymère d'éthylène et d'acide acrylique, ainsi que des combinaisons de ces substances, et dans lequel ledit matériau à base de propylène en phase mésomorphe et ledit copolymère de mélange sont différents afin de former un mélange polymère ;
(b) extruder le mélange polymère ; et
(c) refroidir rapidement le mélange polymère extrudé aussitôt après l'extrusion afin d'obtenir un mélange mésopolymère contenant un matériau à base de propylène globalement en phase mésomorphe, ledit refroidissement rapide se faisant à une température inférieure à 38 °C.

25. Procédé de préparation d'un mélange mésopolymère selon la revendication 24, qui consiste en outre à mélanger du polybutylène avec le copolymère de mélange et le matériau à base de propylène afin d'obtenir le mélange polymère.

26. Procédé de préparation d'un mélange mésopolymère selon l'une quelconque des revendications 24 à 25 précédentes, qui consiste en outre, après l'étape de refroidissement rapide, à exposer le mélange mésopolymère à une dose de rayonnement ionisant comprise entre 1 kGy et 200 kGy.

27. Procédé de préparation d'un mélange mésopolymère selon l'une quelconque des revendications 24 à 26 précédentes, qui consiste en outre, après l'étape de refroidissement rapide, à greffer une couche greffée sur la surface du mélange mésopolymère en l'exposant à une dose de rayonnement ionisant.

28. Mélange polymère selon la revendication 27, dans lequel le rayonnement ionisant comprend un rayonnement par faisceau d'électrons compris entre 5 kGy et 200 kGy.

29. Procédé de préparation d'un mélange mésopolymère selon l'une quelconque des revendications 27 à 28, dans lequel la couche greffée améliore une ou plusieurs propriétés du mélange polymère, notamment l'adhérence en surface, le coefficient de frottement, la perméabilité à l'oxygène, la perméabilité à l'humidité, ou des combinaisons de celles-ci.

30. Article formé à partir d'un mélange mésopolymère comprenant un matériau à base de propylène globalement en phase mésomorphe et au moins un pour cent en poids d'un copolymère de mélange choisi dans le groupe comprenant un mésocopolymère, un copolymère d'éthylène et d'acétate de vinyle, un copolymère d'éthylène et d'acide acrylique, ainsi que des combinaisons de ces substances, et dans lequel ledit matériau à base de propylène en phase mésomorphe et ledit copolymère de mélange sont différents et dans lequel ledit mélange à l'état fondu a été rapidement refroidi afin d'obtenir une phase mésomorphe.

31. Article selon la revendication 30, dans lequel l'article comprend un film, une fibre, une microfibre, un tube ou une poche.

32. Article selon l'une quelconque des revendications précédentes 30 à 31, qui comprend en outre une couche greffée fixée sur la surface de l'article par une dose de rayonnement ionisant.

33. Article selon la revendication 32, dans lequel la couche greffée améliore une ou plusieurs propriétés de l'article, notamment l'adhérence en surface, le coefficient de frottement, la perméabilité à l'oxygène, la perméabilité à l'humidité, ou des combinaisons de celles-ci.

34. Article selon la revendication 33, dans lequel la couche greffée comprend une couche d'adhérence en surface formée en fixant sur l'article un composé choisi dans le groupe comprenant l'acide acrylique, le diméthylacrylamide, la N-vinyl-2-pyrrolidone, un copolymère de N-vinyl-2-pyrrolidone et de triacrylate de triméthylolpropane, l'acrylate de glycidyle, l'acrylate d'hydroxyéthyle, l'acrylate d'hydroxyméthyle, la 2-vinylpyridine, le méthacrylate de sulfoéthyle, le diisopropylacrylamide, le N,N-diéthylaminoacrylate, ainsi que des combinaisons de ces substances.

35. Utilisation d'un article formé à partir d'un mélange mésopolymère qui consiste à :
(a) fournir un article selon la revendication 30 ; et
(b) interposer ledit article entre un environnement protégé et un environnement externe de façon à ce que l'environnement protégé reste sensiblement exempt de contamination.

36. Utilisation selon la revendication 35, dans laquelle l'article comprend un film d'emballage contenant un produit dégradable dans l'environnement protégé.

37. Utilisation selon la revendication 36, dans laquelle le produit dégradable comprend un produit alimentaire ou un produit pharmaceutique.

38. Utilisation selon la revendication 35, dans laquelle l'article comprend un timbre transdermique de délivrance de médicaments, une bande médicale, une tubulure médicale ou une poche d'ostomie.

39. Utilisation selon la revendication 35, dans laquelle l'article comprend en outre une couche greffée fixée sur une surface de l'article par une dose de rayonnement ionisant.
